(19)

Europäisches
Patentamt
European
Patent Office
Office européen
des brevets

(11)  **EP 3 856 900 B1**

(12)  # EUROPEAN PATENT SPECIFICATION

(45) Date of publication and mention
of the grant of the patent:
**25.09.2024  Bulletin 2024/39**

(21) Application number: **19867569.6**

(22) Date of filing: **26.09.2019**

(51) International Patent Classification (IPC):
*C12N 11/00* (2006.01)       *C12N 11/02* (2006.01)
*C12N 11/08* (2020.01)       *C12N 11/14* (2006.01)
*H01F 1/00* (2006.01)       *B22F 10/20* (2021.01)
*B33Y 70/10* (2020.01)       *B33Y 80/00* (2015.01)
*B33Y 10/00* (2015.01)       *C12N 9/02* (2006.01)
*C12N 9/08* (2006.01)       *C12N 9/20* (2006.01)
*C12N 9/26* (2006.01)       *C12N 9/90* (2006.01)
*C12N 11/082* (2020.01)       *C12N 11/098* (2020.01)
*C22C 1/04* (2023.01)

(52) Cooperative Patent Classification (CPC):
**C12N 11/098; B33Y 10/00; B33Y 70/10;
B33Y 80/00; C12N 9/0042; C12N 9/0065;
C12N 9/0071; C12N 9/20; C12N 9/2431;
C12N 9/90; C12N 11/08; C12N 11/082;
C12N 11/14; C22C 1/0441; H01F 1/0063;**    (Cont.)

(86) International application number:
**PCT/US2019/053307**

(87) International publication number:
**WO 2020/069227 (02.04.2020 Gazette 2020/14)**

(54) **PRINTABLE MAGNETIC POWDERS AND 3D PRINTED OBJECTS FOR BIONANOCATALYST
IMMOBILIZATION**

BEDRUCKBARE MAGNETISCHE PULVER UND 3D-BEDRUCKTE OBJEKTE FÜR DIE
IMMOBILISIERUNG VON BIONANOKATALYSATOREN

POUDRES MAGNÉTIQUES IMPRIMABLES ET OBJETS IMPRIMÉS PAR IMPRESSION 3D POUR
IMMOBILISATION DE BIO-NANOCATALYSEURS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **27.09.2018   US 201862737910 P**

(43) Date of publication of application:
**04.08.2021   Bulletin 2021/31**

(73) Proprietor: **ZYMtronix Catalytic Systems, Inc.**
**Ithaca, NY 14853 (US)**

(72) Inventors:
• **CORGIE, Stephane Cedric**
**Ithaca, New York 14853 (US)**

• **CHUN, Matthew Stephen**
**Ithaca, New York 14853 (US)**
• **RIVERA, Katia Argelia Rodriguez**
**Ithaca, New York 14853 (US)**
• **SANKTJOHANSER, Maximilian Josef**
**Ithaca, New York 14853 (US)**
• **WONG, Braedon Carter**
**Ithaca, New York 14853 (US)**

(74) Representative: **Kilburn & Strode LLP**
**Lacon London**
**84 Theobalds Road**
**Holborn**
**London WC1X 8NL (GB)**

(56) References cited:
**EP-A1- 3 159 141       WO-A1-2017/011292**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**WO-A1-2017/180383      CA-A1- 3 020 916**
**US-A1- 2017 189 960**

(52)  Cooperative Patent Classification (CPC): (Cont.)
       B22F 10/18; B22F 10/28; B22F 10/34

**Description**

## CROSS REFERENCE TO RELATED APPLICATIONS

This application claims the benefit of U.S. Provisional Application No. 62/737,910, filed September 27, 2018.

## FIELD OF THE INVENTION

The invention provides materials, and in particular, magnetic materials, for the universal immobilization of enzymes and enzyme systems. Described herein are high magnetism and high porosity composite blends of thermoplastics with magnetic particles to form powders, single-layered, or multiple-layered materials that are used as scaffolds for magnetically immobilized enzymes known as bionanocatalysts (BNCs). Designed objects are produced using 3D printing by sintering composite magnetic powders. In some embodiments, Selective Laser Sintering (SLS) is used. The invention provides the use of the composition materials for 3D printing of enzyme supports and flow cells allowing continuous production of small molecules.

## BACKGROUND OF THE INVENTION

Biocatalysis, as a green technology, has become increasingly popular in chemical manufacturing over traditional expensive and inefficient processes. Its applications include the production of food ingredients, flavors, fragrances, commodity and fine chemicals, and active pharmaceuticals. When producing chemicals at industrial scale, however, enzymes can suffer drastic losses in activity and loading causing a significant drop in performance.

Magnetic enzyme immobilization involves the entrapment of enzymes in mesoporous magnetic clusters that self-assemble around the enzymes (level 1). The immobilization efficiency depends on a number of factors that include the initial concentrations of enzymes and nanoparticles, the nature of the enzyme surface, the electrostatic potential of the enzyme, the nature of the nanoparticle surface, and the time of contact. Enzymes used for industrial purposes in biocatalytic processes should be highly efficient, stable before and during the process, reusable over several biocatalytic cycles, and economical.

Mesoporous aggregates of magnetic nanoparticles may be incorporated into continuous or particulate macroporous scaffolds (level 2). The scaffolds may or may not be magnetic. Such scaffolds are discussed in WO2014/055853, WO2017/180383, and Corgie et al., Chem. Today 34(5):15-20 (2016). Highly magnetic scaffolds are designed to immobilize, stabilize, and optimize any enzyme. This includes full enzyme systems, at high loading and full activity, and for the production of, *e.g.,* small molecules. EP 3159141 describes a magnetic microporous powder for 3D printing, comprising thermoplastic polymer and magnetic microparticles.

Selective laser sintering (SLS) is an additive manufacturing (AM) technique that uses a laser as the power source to sinter powdered materials such as nylon or polyamide. A laser automatically aimed at points in space, defined by a 3D model, binds the material together to create a solid structure. It is similar to direct metal laser sintering (DMLS) but differs in technical details. DMI,S uses a comparable concept, but in DMLS the material is fully melted rather than sintered. This allows one to manufacture materials with different properties *(e.g.* crystal structure and porosity). SLS is a relatively new technology that may be expanded into commercial-scale manufacturing processes.

SLS is an additive manufacturing layer technology. It involves high-powered lasers *(e.g.* a carbon dioxide laser) to fuse small particles of plastic, metal, ceramic, or glass powders into a mass that has a desired three-dimensional shape. The laser selectively fuses powdered material by scanning cross-sections generated from a 3-D digital description of the part (for example from a CAD file or scan data) on the surface of a powder bed. After each cross-section is scanned, the powder bed is lowered by a one-layer thickness, a new layer of material is applied on top, and the process is repeated until the part is completed.

3D printing involves processes in which material is joined or solidified under computer control to create three-dimensional objects. Material is added together (e.g. liquid molecules or powder grains) that is fused together. 3D printing is used in both rapid prototyping and additive manufacturing. Objects can be of almost any shape or geometry and typically are produced using digital model data from a 3D model or another electronic data source such as an Additive Manufacturing File (AMF). Typically, materials are added in sequential layers. There are many different technologies, like stereolithography (SLA) or fused deposit modeling (FDM).

## SUMMARY OF THE INVENTION

Aspects of the invention for which protection is sought are as defined in the claims. According to a first aspect of the invention, there is provided a shaped magnetic macroporous scaffold formed into said shape by three-dimensional (3D) printing; wherein said shaped magnetic macroporous scaffold comprises:

a. a magnetic macroporous powder comprising a thermoplastic polymer and magnetic microparticles;
b. self-assembled mesoporous aggregates of magnetic nanoparticles; and

i. optionally wherein said self-assembled mesoporous aggregates of magnetic nanoparticles further comprise one or more enzymes magnetically immobilized within said mesopores or on the surface of said magnetic nanoparticles;
ii. optionally wherein said magnetic particles have a size of: between about 50-100$\mu$m, between about 10-50$\mu$m, between about 5-10$\mu$m, about 10$\mu$m, about 5$\mu$m, less than about 5$\mu$m, or greater than about 100$\mu$m;
iii. optionally wherein said magnetic macroporous powder has an average size of about 150 $\mu$m, 75 $\mu$m or 15 $\mu$m; or
iv. optionally wherein said magnetic particles have a concentration of between 0 and 10% by weight, 10 to 50% by weight, or 50 to 90% by weight.

According to a second aspect, there is provided a method of making the shaped magnetic macroporous scaffold of the first aspect of the invention, comprising additively manufacturing (AM) said shaped magnetic macroporous scaffold using a three-dimensional (3D) printer, wherein said shape is taken from a 3D model;

a. optionally wherein said 3D model is an electronic file;
b. optionally wherein said electronic file is a computer-aided design (CAD) or a stereolithography (STL) file;
c. optionally wherein said AM is Fused Filament Fabrication (FFF) or Selective laser sintering (SLS); or
d. optionally wherein said macropores are formed using a soluble agent selected from the group consisting of a salt, a sugar, or a small soluble polymer, and removing said soluble agent with a solvent.

According to a third aspect of the invention, there is provided a method of making a device for catalyzing an enzymatic reaction, comprising combining the shaped magnetic macroporous scaffold of the first aspect of the invention and an enzyme, wherein said enzyme is magnetically immobilized within said mesopores.

The invention employs a magnetic macroporous powder, comprising a thermoplastic polymer and magnetic microparticles, wherein the powder is operative for immobilizing self-assembled mesoporous aggregates of magnetic nanoparticles.

In some embodiments, the magnetic particles have a size of between about 50-100$\mu$m, 10-50$\mu$m, 5-10$\mu$m, 10$\mu$m, or 5$\mu$m. In other embodiments, the magnetic particles have a size of less than 5$\mu$m, greater than 100$\mu$m or have an average size of about 150 $\mu$m, about 75 $\mu$m, or about 15 $\mu$m.

In some embodiments, the magnetic particles have a concentration of between 0 and 10% by weight. In other embodiments, the magnetic particles have a concentration of 10 to 50% or 50 to 90% by weight.

In some embodiments, the thermoplastic polymer comprises a polymer selected from the group consisting of Polyvinyl alcohol (PVA), Acrylic (PMMA), Acrylonitrile butadiene styrene (ABS), Polyamide including Nylon 6, Nylon 11 and Nylon 12, Polylactic acid (PLA), Polybenzimidazole (PBI), Polycarbonate (PC), Polyether sulfone (PES), Polyoxymethylene (POM), Polyetherether ketone (PEEK), Polyetherimide (PEI), Polyethylene (PE), Polyphenylene oxide (PEO), Polyphenylene sulfide (PPS), Polypropylene (PP), Polystyrene (PS), Polyvinyl chloride (PVC), polytetrafluoroethylene (PTFE), co-polyesters, and chemically functionalized derivatives thereof.

In some embodiments, the magnetic microparticles comprise a magnetic material selected from the group consisting of magnetite ($Fe_3O_4$), hematite ($\alpha$-$Fe_2O_3$), maghemite ($\gamma$-$Fe_2O_3$), a spinel ferrite, lodestone, cobalt, nickel, rare earth, and magnetic composites. In preferred embodiments, the rare earth is neodymium, gadolinium, sysprosium, samarium-cobalt, or neodymium-iron-boron. In other embodiments, the magnetic composite comprises a ceramic, ferrite, or alnico magnet.

The invention provides that, in the magnetic macroporous powders disclosed herein, the thermoplastic polymer and the magnetic microparticles are chemically, thermally, or physically blended.

In some embodiments, the macropores have a size of between 0.5-200$\mu$m.

In some embodiments, the macroporous powders of the invention further comprise cellulose fibers, cellulose nanofibers, glass fibers, or carbon fibers or other reinforcement macrostructures.

Some embodiments of the magnetic macroporous powder of the invention further comprise self-assembled mesoporous aggregates of magnetic nanoparticles and an enzyme magnetically immobilized within the mesopores or on their surface.

The invention provides shaped magnetic macroporous scaffolds, comprising the magnetic macroporous powders disclosed herein, wherein a powder has been formed into the shape by three-dimensional (3D) printing. In some embodiments, the shape is a cylinder, an orb, a bead, a strip, a capsule, a cube, a squared rod, a pyramid, a diamond, a lattice, or an irregular shape.

In some embodiments, the shaped magnetic macroporous scaffolds described herein further comprise self-assembled mesoporous aggregates of magnetic nanoparticles. In preferred embodiments, the self-assembled mesoporous aggregates of magnetic nanoparticles further comprise one or more enzymes magnetically immobilized within said mesopores or on the surface of said magnetic nanoparticles.

In some embodiments of the invention, the enzyme is selected from the group consisting of hydrolases, hydroxylases, hydrogen peroxide producing enzymes (HPP), nitralases, hydratases, dehydrogenases, transaminases, ketoreductases (KREDS) ene reductases (EREDS), imine reductases (IREDS), catalases, dismutases, oxidases, dioxygenases, lipoxidases, oxidoreductases, peroxidases, laccases, synthetases, transferases, oxynitrilases, isomerases, gludosidases, kinases, lyases, sucrases, invertases, epimerases, and lipases. In some embodiments of the invention, two or more enzymes are combined.

In other embodiments of the invention, the self-assembled mesoporous aggregates of magnetic nanoparticles comprise microsomes, wherein a first enzyme requiring a diffusible cofactor having a first enzymatic activity is contained within the microsomes, wherein a second enzyme comprising a cofactor regeneration activity is magnetically-entrapped within the mesopores, wherein the cofactor is utilized in the first enzymatic activity; wherein the first and second enzymes function by converting a diffusible substrate into a diffusible product; and wherein the magnetic nanoparticles are magnetically associated with the magnetic macroporous scaffold.

In other embodiments of the invention, the self-assembled mesoporous aggregates of magnetic nanoparticles comprises a first enzyme requiring a diffusible cofactor having a first enzymatic activity; a second enzyme comprising a cofactor regeneration activity; wherein the cofactor is utilized in the first enzymatic activity; wherein the first and second enzymes are magnetically-entrapped within the mesopores formed by the aggregates of magnetic nanoparticles and the first and second enzymes function by converting a diffusible substrate into a diffusible product. In preferred embodiments, the first enzyme is an oxidative enzyme. In more preferred embodiments, the oxidative enzyme is a Flavin-containing oxygenase and further comprising a third enzyme having a co-factor reductase activity that is co-located with the first enzyme.

In a more preferred embodiment, the oxidative enzyme is a P450 monooxygenase and the composition further comprises a third enzyme having a co-factor reductase activity that is co-located with the first enzyme. In a more preferred embodiment, the P450 monooxygenase and the third enzyme are comprised within a single protein. In a more preferred embodiment, the single protein comprises a bifunctional cytochrome P450/NADPH--P450 reductase. In a more preferred embodiment, the single protein has BM3 activity and has at least a 90% sequence identity to SEQ ID NO: 1.

In some embodiments of the invention, the macroporous scaffolds disclosed herein are formed in a shape suited for a particular biocatalytic process.

The invention provides a method of making a shaped magnetic macroporous scaffold comprising the magnetic macroporous powders disclosed herein, comprising additively manufacturing (AM) the shaped magnetic macroporous scaffold using a three-dimensional (3D) printer, wherein the shape is taken from a 3D model. In some embodiments, the 3D model is an electronic file. In preferred embodiments, the electronic file is a computer-aided design (CAD) or a stereolithography (STL) file.

In some embodiments, the AM is Fused Filament Fabrication (FFF) or Selective Laser Sintering (SLS).

In some embodiments of the invention, the macropores are formed using a soluble agent selected from the group consisting of a salt, a sugar, or a small soluble polymer. The soluble agent is removed with a solvent.

The invention provides a method of making a device for catalyzing an enzymatic reaction, comprising combining a shaped magnetic macroporous scaffold with self-assembled mesoporous aggregates of magnetic nanoparticles and an enzyme, wherein the enzyme is magnetically immobilized within the mesopores.

The invention provides a method of catalyzing a reaction between a plurality of substrates, comprising exposing the magnetic macroporous powders disclosed herein comprising an enzyme to the substrates under conditions in which the enzyme catalyzes the reaction between the substrates.

The invention provides a method of catalyzing a reaction between a plurality of substrates, comprising exposing the shaped magnetic macroporous scaffold disclosed herein comprising an enzyme to the substrates under conditions in which the enzyme catalyzes the reaction between the substrates.

In other embodiments, the reaction is used in the manufacture of a pharmaceutical product, a medicament, a food product, a flavor, a fragrance, a sweetener, an agrochemical, an antimicrobial agent, a toxin, a detergent, a fuel product, a biochemical product, a paper product, or a plastic product. In other embodiments, the reaction is used in a process for removing a contaminant from a solution. In preferred embodiments, the solution is an aqueous solution, a solvent, or an oil.

In some embodiments, the methods of manufacturing disclosed herein further comprise the step of removing the mesoporous aggregates and replacing them with a fresh preparation of mesoporous aggregates. In other embodiments, the method is carried out using flow cell and continuous manufacturing.

## BRIEF DESCRIPTION OF THE DRAWINGS

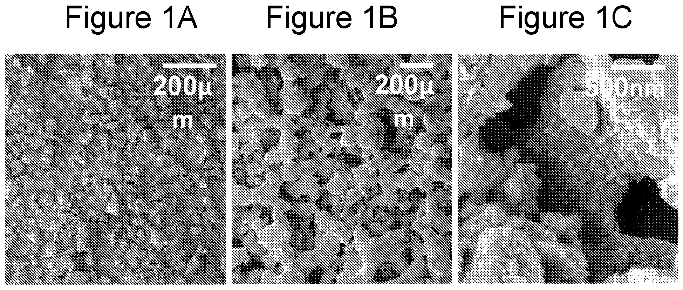

SEM images of a polypropylene magnetite composite extruded composite after: **(Figure 1A)** cryogenic size reduction and sieving through a 100 micron opening; **(Figure 1B)** selective laser sintering at a chamber temperature of 105°C, surface temperature of 125°C, and a laser speed of 650 mm/s; and **(Figure 1C)** enzyme immobilization of the sintered scaffold (cross) showing the BNCs (invertase enzyme) magnetically captured on the surface.

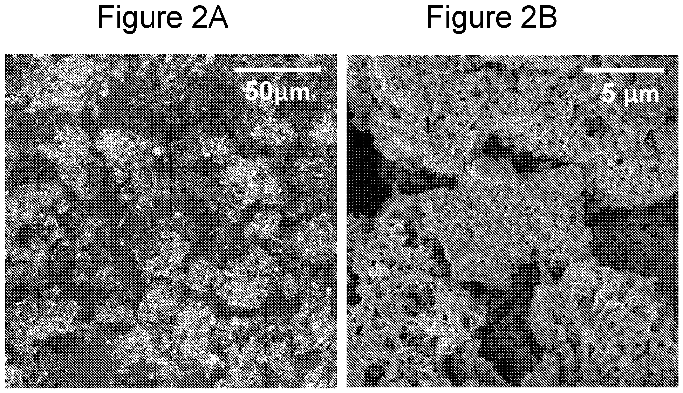

SEM images of a Nylon 12 magnetite composite produced through a dissolution coating process. **Figure 2A** shows a backscatter detector. **Figure 2B** shows it at 10,000 times magnification.

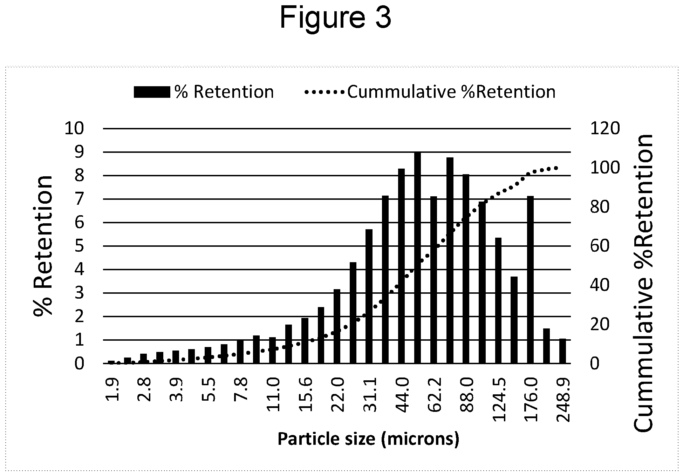

**Figure 3** shows an article size analysis of a polypropylene magnetite extruded composite after cryogenic size reduction.

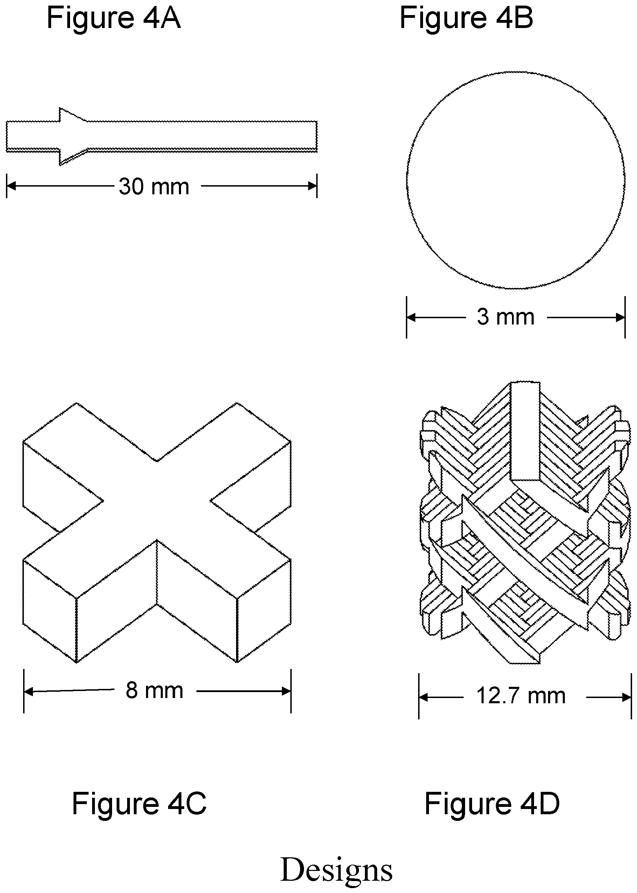

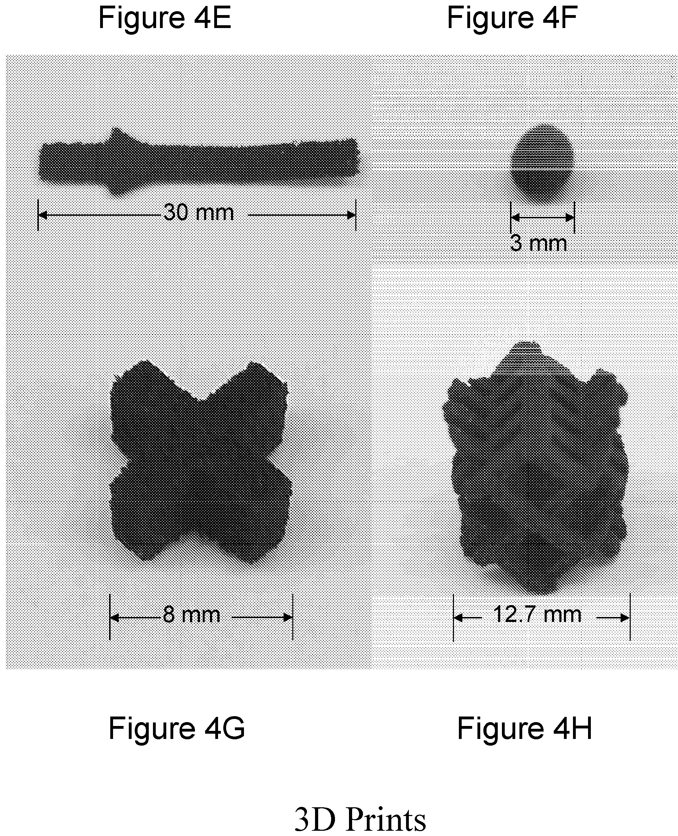

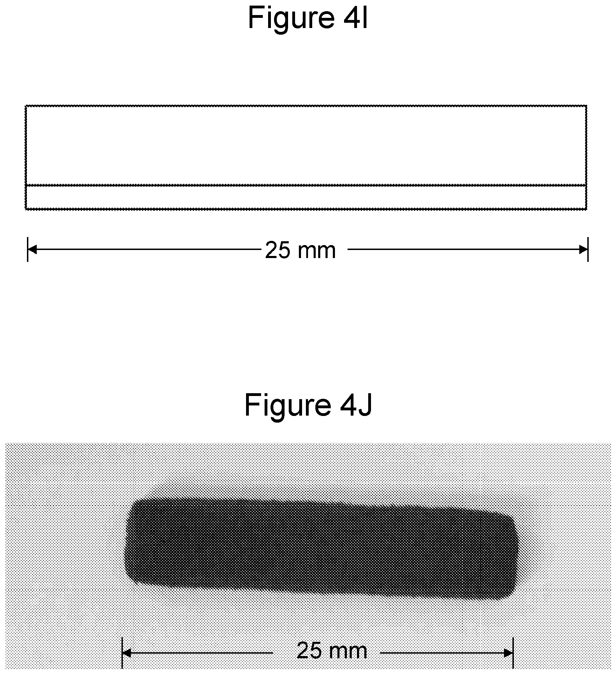

Sintering of 3D printing objects for enzyme immobilization. 3D renderings are shown for a strip **(Figure 4A),** a bead **(Figure 4B),** a cross **(Figure 4C),** and a static mixing element **(Figure 4D).** The corresponding SLS printed objects are shown in **Figures 4E, 4F, 4G,** and **4H.** They were printed using a 110°C chamber temperature, 125°C fabrication surface temperature, and a 650mm/s laser speed. **Figure 4I** depicts a 3D render of a printed bar for enzyme immobilization. **Figure 4J** depicts the SLS printed bar.

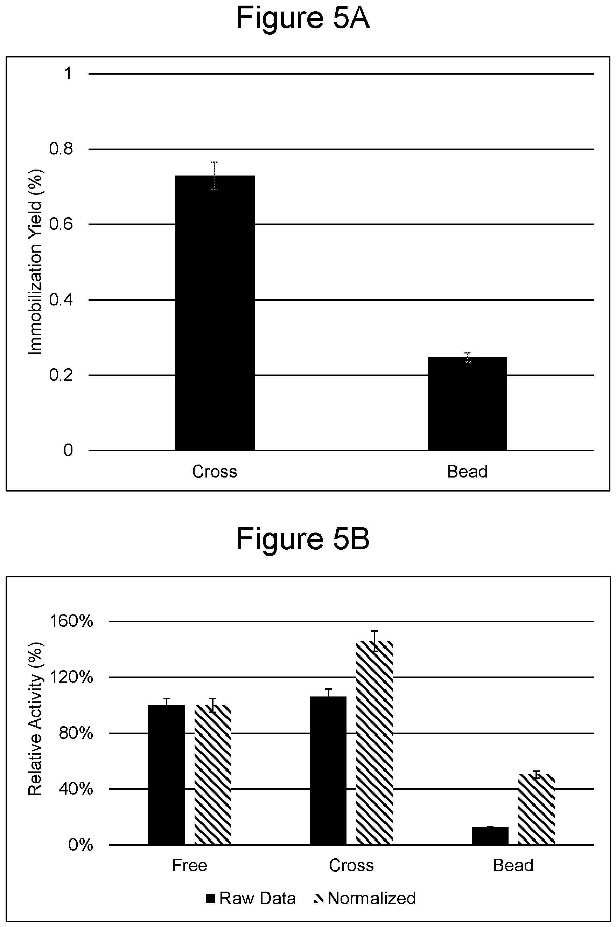

Immobilization of invertase. **Figure 5A** shows the immobilization yields of invertase on 3D printed polypropylene-magnetite scaffolds (Polypropylene-magnetite cross and bead). Immobilization conditions: 500 $\mu$g/mL NP pH 3. **Figure 5B** shows the relative activity of free and immobilized invertase (100 $\mu$g/mL) for the hydrolysis of raffinose after 5 minutes, as determined by the reducing-sugar PAHBAH assay.

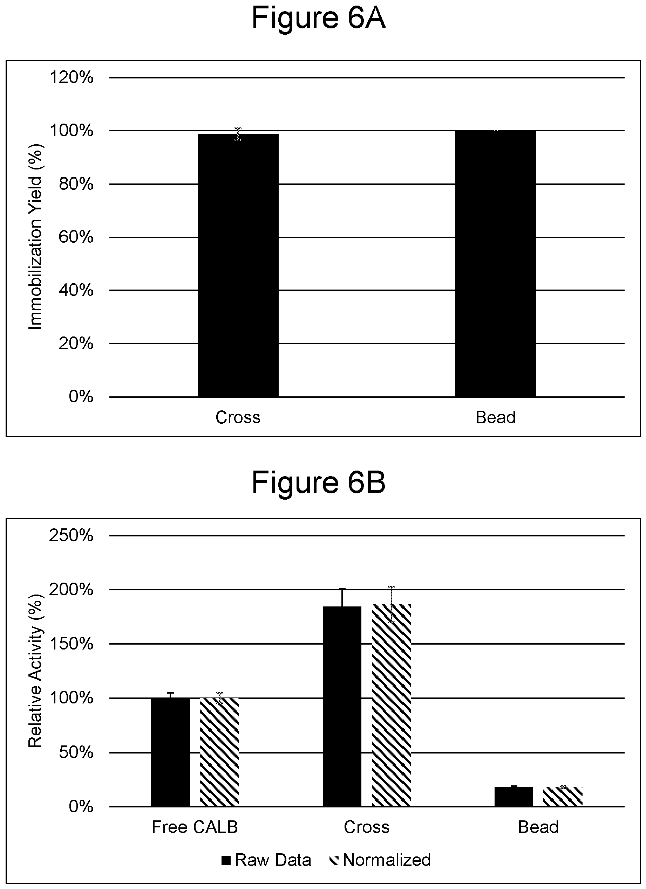

Immobilization of lipase. **Figure 6A** shows the immobilization yields of CALB on 3D printed scaffolds (Polypropylene-magnetite cross and bead). Immobilization conditions: 250 $\mu$g/mL NP pH 3. **Figure 6B** shows the relative activity of free and immobilized CALB (100 $\mu$g/mL) for the hydrolysis of p-NPL after 4 minutes as determined by colorimetric assay.

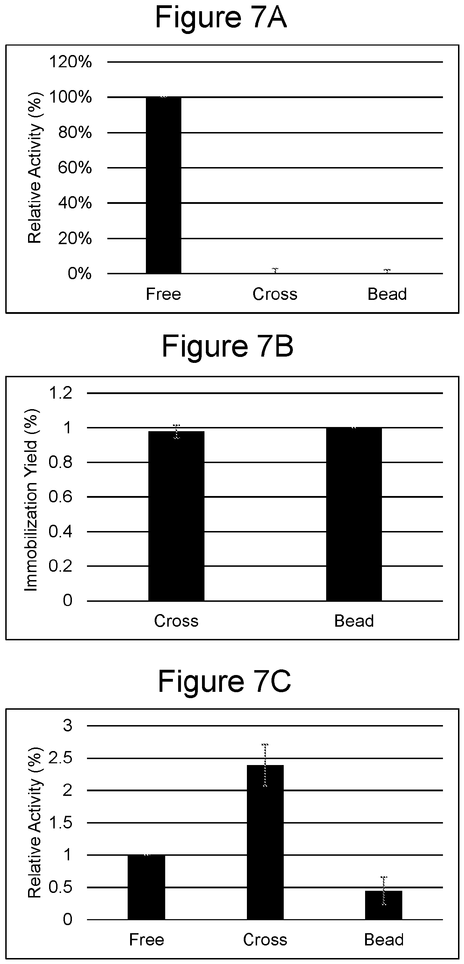

Re-use of the 3D printed scaffolds on a polypropylene-magnetite cross and beads. **Figure 7A** shows the relative activity of free CALB (100 $\mu$g/mL) and previously immobilized crosses and beads (post-wash in boiling pH 2 water for 10 mins) for the hydrolysis of p-NPL after 4 minutes as determined by colorimetric assay. **Figure 7B** shows the re-immobilization yields of CALB on 3D printed scaffolds (cross and bead). Immobilization conditions were 250 $\mu$g/mL NP at pH 3. **Figure 7C** shows the relative activity of free CALB and re-immobilized CALB on washed scaffolds as determined by p-NPL hydrolysis.

**Figure 8** shows a plot of a flow-rate, F, (mL/min) from a sucrose reaction mixture in a flow cell of immobilized invertase (modeled as a PBR) as a function of various initial sucrose concentrations, $S_o$. It also shows the conversion of sucrose into glucose and fructose, X.

**Figure 9** shows the relative activity of immobilized HRP to free enzyme normalized for immobilization yield for the co-oxidation of 4-AAP and phenol as determined by colorimetric assay.

**Figure 10:** A 3D printed flow cell set up with a syringe pump. HRP was immobilized onto the flow cell and the activity was confirmed by the colorimetric assay for co-oxidation of 4-AAP and phenol.

## DETAILED DESCRIPTION OF THE INVENTION

The present invention provides a macroporous scaffold and methods for supporting and enhancing the effectiveness of BNC's. This is accomplished, for the first time, using powders, single-layered, and multi-layered forms that are highly magnetic, highly-porous thermoplastic blends. They may be formed using 3D printing that involves sintering composite magnetic powders.

The invention has many benefits over the prior art. It enables the universal immobilization of enzymes for small molecule syntheses. The materials may contain large macropores or a high magnetic surface area for BNC immobilization. Flexible compositions for sintered magnetic scaffolds can be made with any meltable thermoplastics and magnetic material composition. The materials can be made resistant to solvents.

The process is flexible and tunable to manufacture objects using 3D designs that magnetically capture the BNCs. It is scalable from micro-structures to flow cells for biocatalytic reactors. A large surface area may result from the sintering process itself. Materials can also be recycled by removing the BNCs and then re-functionalized them for repeated use.

In some embodiments, thermoplastics such as polypropylene, polyethylene, nylon, and polylactic acid (PLA) are blended with magnetic materials (e.g. magnetite MMP) via melting/extrusion or via coating of the magnetic material by dissolving the plastic in a solvent. In other embodiments, the powders are sintered by a laser using SLS. Porosity may be formed during SLS.

In other embodiments, an extruded composite material is size reduced via cryomilling or another form of milling.

In other embodiments, composite powders are sieved to an ideal particle size. In preferred embodiments, the particle sizes are 60 +/- 20 μm.

Powders or 3D printed objects can be functionalized with BNCs containing one or more enzymes or enzyme systems. BNCs are magnetically trapped at the surface of the powders or 3D printed objects.

Composite powders may also be optimized for flowability. In some embodiments, 3D objects can be printed to optimize flow within to be used in flow reactors.

In some embodiments, 3D objects and composite powders can be washed from the BNCs by an acid wash, rinsed with water, and then re-functionalized with fresh BNCs.

Highly magnetic scaffolds (Macroporous Magnetic Scaffolds or MMP) are designed to immobilize, stabilize and optimize any BNCs containing enzymes. This includes full enzyme systems at high loading and full activity for the production of small molecules. By combining natural or engineered enzymes with cofactor recycling systems, the scaffolds allow one to scale up biocatalysis to innovations to manufacturing scale and production.

MMP made of thermoplastic and magnetic materials can take the form of magnetic powders that are suitable for use in sequential batch reactions. For flow chemistry application, these powders can be 3D printed by SLS as structures, as functional objects, or as flow cells or plate reactors. High surface areas allow one to maximize the enzyme loading and flow can be engineered within the materials to enable biocatalysis at maximal productivity.

SLS can be used to process nearly any kind of material from metals, ceramics, plastics, and combinations thereof, for tailor-made composite materials. It is critical, however, that the material is available in fine powder form and that the powder particles are operative to fuse when exposing them to heat (Kruth et al., Assembly Automation 23(4):357-371 (2003)).

When the material lacks those features, or is prone to phase transitions at the temperature range or conditions of the sintering process, the addition of a sacrificial binder can make this process still feasible for that material. Commonly, polymers are used as sacrificial binders in order to expand the range of materials suitable for this technology. After sintering, the sacrificial binder can be either removed by thermal decomposition or kept as part of the composition.

This concept applies to magnetite that loses its permanent magnetic properties above 585°C. This is significantly lower than its melting temperature (1538°C). Another advantage of using a polymeric matrix to incorporate magnetite particles is that the former can act as a protective barrier to prevent oxidation and corrosion as well as aiding to disperse the magnetite particles. Also, magnetite can mechanically reinforce the polymer. (Shishkovsky et al., Microelectronic Engineering 146:85-91 (2015)).

Laser sintering of plastic parts is one of two additive manufacturing processes used for Rapid Manufacturing (Wegner, Physics Procedia 83:1003-1012 (2016)). There are several polymer properties that determine its capability to be sintered and produce good quality 3D objects. These include structural properties such crystalline structure (*i.e.* thermal properties such as Tm, Tg, and Tc), mechanical properties (Young's modulus and elongation at break, etc.), density, particle size, and shape.

In SLS, the temperature-processing window is determined from the difference between the melting and crystallization temperatures of the polymer. For instance, nylon 12 (PA 12) has one of the highest operational windows and is thus a widely used SLS material. In theory, the higher this value is, the easier the material can be sintered. In practice, however there are many more parameters that can still make this process difficult for any specific polymer (Shishkovsky et al., Microelectronic Engineering 146:85-91 (2015)). In order to prevent curling of the sintered part, a low polymer crystallization rate is desired together with a melt index that provides a suitable rheology and surface tension.

Additionally, the bulk density, particle shape, and size distribution of the powder are key factors (Wegner, Physics Procedia 83:1003-1012 (2016)). It has been determined that the optimal particle size range is about 40 to about 90 microns. Smaller particles prevent flowability and their rapid vaporization is detrimental to the optical sensors of the sintering device. This can fog the device and lead to inaccurately sintered parts (Goodridge et al. Materials Science 57:229-267 (2012)). The powders should have good flowing properties and preferably an approximately round particle shape. This allows good powder spreading during the process. High heat conductivity of the material is desired at the $CO_2$ laser beam wavelength (10.6 microns). This is not the case for most polymers. The last two requirements can be met by the incorporation of additives such as high-energy absorption materials, e.g. carbon black, to improve heat absorption, and fume silica nanoparticles (talc) to aid the particle flowability with irregularly-shaped particles.

Additive manufacturing (AM), also referred to as 3D printing, involves manufacturing a part by depositing material layer-by-layer. This differs from conventional processes such as subtractive processes (*i.e.,* milling or drilling), formative processes (*i.e.,* casting or forging), and joining processes (*i.e.,* welding or fastening). Quick production time, low prototyping costs, and design flexibility make 3D printing a valuable tool for both prototyping and industrial manufacturing. The three most common types of 3D printers are fused filament fabrication, stereolithography, and selective laser sintering.

Fused filament fabrication (FFF) melts a thermoplastic continuous filament and builds the object layer by layer until the print is complete. Although alternative materials exist, the two most popular filament materials are polylactic acid (PLA) and acrylonitrile butadiene styrene (ABS). FFF printers and materials are among the cheapest on the market but currently have a lower print resolution and build quality.

Stereolithography (SLA) uses a laser to polymerize photosensitive resins. Uncured liquid resin is placed in a vat where a laser is used to cure resin into solid plastic and build the object layer by layer. SLA printers have a much higher resolution than FFF printers due to the fine spot size of the laser and thus can print intricate features and complex shapes. The resins, however, are more expensive than filaments and completed prints currently require post processing with solvents to optimize the surface finish and material characteristics.

Selective laser sintering (SLS) is a powder-based layer-additive manufacturing process generally meant for rapid prototyping and rapid tooling. Laser beams either in continuous or pulse mode are used as a heat source for scanning and joining powders in predetermined sizes and shapes of layers. The geometry of the scanned layers corresponds to the various cross sections of the computer-aided design (CAD) models or stereolithography (STL) files of the object. After the first layer is scanned, a second layer of loose powder is deposited over it, and the process is repeated from bottom to top until the artifact (3D object) is complete." (Kumar, JOM, 55(10), 43-47 (2003)).

SLS provides advantages for printing objects with magnetic properties that can be used for immobilizing BNCs. This is because the printing process creates porosity and a high surface area. The surrounding, unsintered powder acts as a natural support that eliminates the need for dedicated support structures. The lack of support structures allows for complex geometries that would otherwise be impossible to manufacture using alternative 3D printing methods. In addition, the nature of sintering itself creates macro and microporous volumes. During the printing process, the laser flashes thermoplastic crystalline thermoplastic powders (e.g. Polypropylene, polystyrene) between their glass transition temperature and melting temperature to generate stiff parts. By avoiding amorphous behavior with a quick laser scan speed (>100 mm/s), powders are sintered in place to form small bonds amongst themselves. The low-density powders trap air in their structures resulting in remarkable porosity and surface area in three dimensions. These pores increase the surface area for enzyme immobilization.

In recent years, industrial use of enzymes has garnered significant attention due to the wide range of potential manufacturing applications. Using enzymes in industrial processes offers several advantages over conventional chemical methods. This includes high catalytic activity, the ability to perform complex reactions, and promoting greener chemistry by reducing by-products and the need for toxic chemicals (Singh et al., Microbial enzymes: industrial progress in 21st century. 3 Biotech. 6(2): 174 (2016)).

One of the biggest hindrances to widespread biocatalysis use in industrial production is low enzyme stability. This is further hampered by relatively harsh process conditions that can destabilize enzymes and decrease their lifespan (Mohamad et al., Biotechnology, Biotechnological Equipment 29(2):205-220 (2015)). Furthermore, the free enzymes used in these processes are generally lost from the system as waste products and therefore become a high operating cost. The primary solution to these issues is immobilization of enzymes onto scaffolding to enhance their operational stability and catalytic activity. Enzyme immobilization also provides a method for enzyme recovery, making biocatalytic processes more economically feasible.

Currently, biocatalytic processes for industrial production are generally carried out in batch reactors due to their simplicity and ease of operation. Despite the benefits of using batch reactors, continuous flow systems enable higher productivity and better process control (Wiles C et al., Green Chem. (14):38-54 (2012)). The rapid development of flow chemistry in biocatalytic processes has primarily been driven by a growing interest in process intensification and green chemistry. Continuous flow systems facilitate process intensification by decreasing residence times (often from hours to minutes), reducing the size of equipment required, and enabling production volume enhancement (Tamborini et al., Cell. 36(1):73-78 (2018)). From a green chemistry standpoint, these systems offer significant improvements in safety, waste generation, and energy efficiency due to heat management and mixing control (Newman and Jensen, Green Chem. (15):1456-1472 (2013)).

A solution to combining biocatalysis and continuous flow systems is the use of functionalized flow cells. Biocatalytic flow cells are scaffolds containing immobilized enzymes for use in reactors such as continuous stirred tank reactors (CSTRs) and packed bed reactors (PBRs). Both types of reactors are known in the art but are primarily chosen based on the type of immobilization used. With a total market value of $5.8B in 2010, immobilized enzymes are used in a diverse range of large-scale processes including high fructose corn syrup production ($10^7$ tons/year), transesterification of food oils ($10^5$ tons/year), biodiesel synthesis ($10^4$ tons/year), and chiral resolution of alcohols and amines ($10^3$ tons/year) (DiCosimo et al., Chem. Soc. Rev. (42):6437-6474 (2013)). These systems allow for improved downstream process management for enzymatic systems compared to batch reactors in terms of in-line control, enzyme reuse, and production scalability.

For the foregoing reasons, the inventions described herein provide biocatalytic systems for small-to-large scale manufacturing using BNCs in scaffolds that are shaped by 3D printing. In some embodiments, the biocatalytic systems are continuous flow.

The novel scaffolds disclosed herein may comprise cross-linked water-insoluble polymers and an approximately uniform distribution of embedded magnetic microparticles (MMP). The cross-linked polymer comprises polyvinyl alcohol (PVA) and optionally additional polymeric materials. The scaffolds may take any shape by using 3D printing. By combining natural or engineered enzymes with cofactors and cofactor recycling systems, the scaffold technology disclosed herein allows one to quickly translate innovation in biocatalysis to innovation in production. The magnetic powders are suitable for use in sequential batch reactions. For flow chemistry applications, these powders can be 3D printed as structures in a functional object or as flow cells or plate reactors. High surface areas allow one to maximize the enzyme loadings and flow can be engineered within the materials to enable biocatalysis at maximal productivity.

With the ability to immobilize any enzymes for any processes, the materials functionalized with enzymes, or enzyme systems, have applications for the production of pharmaceutical actives, agricultural actives, flavors and fragrances, fine and commodity chemicals, and food ingredients.

Self-assembled mesoporous nanoclusters comprising entrapped enzymes are highly active and robust. The technology is a powerful blend of biochemistry, nanotechnology, and bioengineering at three integrated levels of organization: Level 1 is the self-assembly of enzymes with magnetic nanoparticles (MNP) for the synthesis of magnetic mesoporous nanoclusters. This level uses a mechanism of molecular self-entrapment to immobilize and stabilize enzymes. Level 2 is the stabilization of the MNPs into other matrices. Level 3 is product conditioning and packaging for Level 1+2 delivery. The assembly of magnetic nanoparticles adsorbed to enzyme is herein also referred to as a "bionanocatalyst" (BNC).

MNPs allow for a broader range of operating conditions such as temperature, ionic strength and pH. The size and magnetization of the MNPs affect the formation and structure of the NPs, all of which have a significant impact on the activity of the entrapped enzymes. By virtue of their surprising resilience under various reaction conditions, MNPs can be used as improved enzymatic or catalytic agents where other such agents are currently used. Furthermore, they can be used in other applications where enzymes have not yet been considered or found applicable.

The BNC contains mesopores that are interstitial spaces between the magnetic nanoparticles. The enzymes are preferably embedded or immobilized within at least a portion of mesopores of the BNC. As used herein, the term "magnetic" encompasses all types of useful magnetic characteristics, including permanent magnetic, superparamagnetic, paramagnetic, ferromagnetic, and ferrimagnetic behaviors.

The magnetic nanoparticle or BNC has a size in the nanoscale, i.e., generally no more than 500 nm. As used herein, the term "size" can refer to a diameter of the magnetic nanoparticle when the magnetic nanoparticle is approximately or substantially spherical. In a case where the magnetic nanoparticle is not approximately or substantially spherical (e.g., substantially ovoid or irregular), the term "size" can refer to either the longest the dimension or an average of the three dimensions of the magnetic nanoparticle. The term "size" may also refer to an average of sizes over a population of magnetic nanoparticles (i.e., "average size").

In different embodiments, the magnetic nanoparticle has a size of precisely, about, up to, or less than, for example, 500 nm, 400 nm, 300 nm, 200 nm, 100 nm, 50 nm, 40 nm, 30 nm, 25 nm, 20 nm, 15 nm, 10 nm, 5 nm, 4 nm, 3 nm, 2 nm, or 1 nm, or a size within a range bounded by any two of the foregoing exemplary sizes.

In the BNC, the individual magnetic nanoparticles can be considered to be primary nanoparticles (i.e., primary crystallites) having any of the sizes provided above. The aggregates of nanoparticles in a BNC are larger in size than the nanoparticles and generally have a size (i.e., secondary size) of at least about 5 nm. In different embodiments, the aggregates have a size of precisely, about, at least, above, up to, or less than, for example, 5 nm, 8 nm, 10 nm, 12 nm, 15 nm, 20 nm, 25 nm, 30 nm, 35 nm, 40 nm, 45 nm, 50 nm, 60 nm, 70 nm, 80 nm, 90 nm, 100 nm, 150 nm, 200 nm, 300 nm, 400 nm, 500 nm, 600 nm, 700 nm, or 800 nm, or a size within a range bounded by any two of the foregoing exemplary sizes.

Typically, the primary and/or aggregated magnetic nanoparticles or BNCs thereof have a distribution of sizes, i.e., they are generally dispersed in size, either narrowly or broadly dispersed. In different embodiments, any range of primary or aggregate sizes can constitute a major or minor proportion of the total range of primary or aggregate sizes. For example, in some embodiments, a particular range of primary particle sizes (for example, at least about 1, 2, 3, 5, or 10 nm and up to about 15, 20, 25, 30, 35, 40, 45, or 50 nm) or a particular range of aggregate particle sizes (for example, at least about 5, 10, 15, or 20 nm and up to about 50, 100, 150, 200, 250, or 300 nm) constitutes at least or above about 50%, 60%, 70%, 80%, 90%, 95%, 98%, 99%, or 100% of the total range of primary particle sizes. In other embodiments, a particular range of primary particle sizes (for example, less than about 1, 2, 3, 5, or 10 nm, or above about 15, 20, 25, 30, 35, 40, 45, or 50 nm) or a particular range of aggregate particle sizes (for example, less than about 20, 10, or 5 nm, or above about 25, 50, 100, 150, 200, 250, or 300 nm) constitutes no more than or less than about 50%, 40%, 30%, 20%, 10%, 5%, 2%, 1%, 0.5%, or 0.1% of the total range of primary particle sizes.

The aggregates of magnetic nanoparticles (i.e., "aggregates") or BNCs thereof can have any degree of porosity, including a substantial lack of porosity depending upon the quantity of individual primary crystallites they are made of. In particular embodiments, the aggregates are mesoporous by containing interstitial mesopores (i.e., mesopores located between primary magnetic nanoparticles, formed by packing arrangements). The mesopores are generally at least 2 nm and up to 50 nm in size. In different embodiments, the mesopores can have a pore size of precisely or about, for example, 2, 3, 4, 5, 10, 12, 15, 20, 25, 30, 35, 40, 45, or 50 nm, or a pore size within a range bounded by any two of the foregoing exemplary pore sizes. Similar to the case of particle sizes, the mesopores typically have a distribution of sizes, i.e., they are generally dispersed in size, either narrowly or broadly dispersed. In different embodiments, any range of mesopore sizes can constitute a major or minor proportion of the total range of mesopore sizes or of the total pore volume. For example, in some embodiments, a particular range of mesopore sizes (for example, at least about 2, 3, or 5, and up to 8, 10, 15, 20, 25, or 30 nm) constitutes at least or above about 50%, 60%, 70%, 80%, 90%, 95%, 98%, 99%, or 100% of the total range of mesopore sizes or of the total pore volume. In other embodiments, a particular range of mesopore sizes (for example, less than about 2, 3, 4, or 5 nm, or above about 10, 15, 20, 25, 30, 35, 40, 45, or 50 nm) constitutes no more than or less than about 50%, 40%, 30%, 20%, 10%, 5%, 2%, 1%, 0.5%, or 0.1% of the total range of mesopore sizes or of the total pore volume.

The magnetic nanoparticles can have any of the compositions known in the art. In some embodiments, the magnetic nanoparticles are or include a zerovalent metallic portion that is magnetic. Some examples of such zerovalent metals include cobalt, nickel, and iron, and their mixtures and alloys. In other embodiments, the magnetic nanoparticles are or include an oxide of a magnetic metal, such as an oxide of cobalt, nickel, or iron, or a mixture thereof. In some embodiments, the magnetic nanoparticles possess distinct core and surface portions. For example, the magnetic nanoparticles may have a core portion composed of elemental iron, cobalt, or nickel and a surface portion composed of a passivating layer, such as a metal oxide or a noble metal coating, such as a layer of gold, platinum, palladium, or silver. In other embodiments, metal oxide magnetic nanoparticles or aggregates thereof are coated with a layer of a noble metal coating. The noble metal coating may, for example, reduce the number of charges on the magnetic nanoparticle surface, which may beneficially increase dispersibility in solution and better control the size of the BNCs. The noble metal coating protects the magnetic nanoparticles against oxidation, solubilization by leaching or by chelation when chelating organic acids, such as citrate, malonate, or tartrate are used in the biochemical reactions or processes. The passivating layer can have any suitable thickness, and particularly, at least, up to, or less than, about for example, 0.1 nm, 0.2 nm, 0.3 nm, 0.4 nm, 0.5 nm, 0.6 nm, 0.7 nm, 0.8 nm, 0.9 nm, 1 nm, 2 nm, 3 nm, 4 nm, 5 nm, 6 nm, 7 nm, 8 nm, 9 nm, or 10 nm, or a thickness in a range bounded by any two of these values.

Magnetic materials useful for the invention are well-known in the art. Non-limiting examples comprise ferromagnetic and ferromagnetic materials including ores such as iron ore (magnetite or lodestone), cobalt, and nickel. In other embodiments, rare earth magnets are used. Non-limiting examples include neodymium, gadolinium, sysprosium, samarium-cobalt, neodymium-iron-boron, and the like. In yet further embodiments, the magnets comprise composite materials. Non-limiting examples include ceramic, ferrite, and alnico magnets. In preferred embodiments, the magnetic nanoparticles have an iron oxide composition. The iron oxide composition can be any of the magnetic or superparamagnetic iron oxide compositions known in the art, e.g., magnetite ($Fe_3O_4$), hematite ($\alpha$-$Fe_2O_3$), maghemite ($\gamma$-$Fe_2O_3$), or a spinel ferrite according to the formula $AB_2O_4$, wherein A is a divalent metal (e.g., $Xn^{2+}$, $Ni^{2+}$, $Mn^{2+}$, $Co^{2+}$, $Ba^{2+}$, $Sr^{2+}$, or combination thereof) and B is a trivalent metal (e.g., $Fe^{3+}$, $Cr^{3+}$, or combination thereof).

The individual magnetic nanoparticles or aggregates thereof or BNCs thereof possess any suitable degree of magnetism. For example, the magnetic nanoparticles, BNCs, or BNC scaffold assemblies can possess a saturated magnetization (Ms) of at least or up to about 5, 10, 15, 20, 25, 30, 40, 45, 50, 60, 70, 80, 90, or 100 emu/g, wherein 1emu/ g=1Am$^2$/kg. The magnetic nanoparticles, BNCs, or BNC-scaffold assemblies preferably possess a permanent magnetization (Mr) of no more than (i.e., up to) or less than 5 emu/g, and more preferably, up to or less than 4 emu/g, 3 emu/g, 2 emu/g, 1 emu/g, 0.5 emu/g, or 0.1 emu/g. The surface magnetic field of the magnetic nanoparticles, BNCs, or BNC-scaffold assemblies can be about or at least, for example, about 0.5, 1, 5, 10, 50, 100, 200, 300, 400, 500, 600, 700, 800, 900, or 1000 Gauss (G), wherein 10$^4$G=1T, or a magnetic field within a range bounded by any two of the foregoing values. If microparticles are included, the microparticles may also possess any of the above magnetic strengths.

The magnetic nanoparticles or aggregates thereof can be made to adsorb a suitable amount of enzyme, up to or below a saturation level, depending on the application, to produce the resulting BNC. In different embodiments, the magnetic nanoparticles or aggregates thereof may adsorb about, at least, up to, or less than, for example, 1, 5, 10, 15, 20, 25, or 30 pmol/m$^2$ of enzyme. Alternatively, the magnetic nanoparticles or aggregates thereof may adsorb an amount of enzyme that is about, at least, up to, or less than, for example, about 10%, 20%, 30%, 40%, 50%, 60%, 70%, 80%, 90%, or 100% of a saturation level.

The magnetic nanoparticles or aggregates thereof or BNCs thereof possess any suitable pore volume. For example, the magnetic nanoparticles or aggregates thereof can possess a pore volume of about, at least, up to, or less than, for example, about 0.01, 0.05, 0.1, 0.15, 0. 2, 0.25, 0.3, 0.35, 0.4, 0.45, 0.5, 0.55, 0.6, 0.65, 0.7, 0.75, 0.8, 0.85, 0.9, 0.95, or 1 cm$^3$/g, or a pore volume within a range bounded by any two of the foregoing values.

The magnetic nanoparticles or aggregates thereof or BNCs thereof possess any suitable specific surface area. For example, the magnetic nanoparticles or aggregates thereof can have a specific surface area of about, at least, up to, or less than, for example, about 50, 60, 70, 80, 90, 100, 110, 120, 130, 140, 150, 160, 170, 180, 190, or 200 m$^2$/g.

MNPs, their structures, organizations, suitable enzymes, and uses are described in WO2012122437 and WO2014055853.

Some embodiments of the invention comprise hydrolases. Hydrolases catalyze the hydrolysis of many types of chemical bonds by using water as a substrate. The substrates typically have hydrogen and hydroxyl groups at the site of the broken bonds. Hydrolases are classified as EC 3 in the EC number classification of enzymes. Hydrolases can be further classified into several subclasses, based upon the bonds they act upon. Exemplary hydrolases and the bonds they hydrolyze include EC 3.1: ester bonds (esterases: nucleases, phosphodiesterases, lipase, phosphatase), EC 3.2: sugars (DNA glycosylases, glycoside hydrolase), EC 3.3: ether bonds, EC 3.4: peptide bonds (Proteases/peptidases), EC 3.5: carbon-nitrogen bonds, other than peptide bonds, EC 3.6 acid anhydrides (acid anhydride hydrolases, including helicases and GTPase), EC 3.7 carbon-carbon bonds, EC 3.8 halide bonds, EC 3.9: phosphorus-nitrogen bonds, EC 3.10: sulphur-nitrogen bonds, EC 3.11: carbon-phosphorus bonds, EC 3.12: sulfur-sulfur bonds, and EC 3.13: carbon-sulfur bonds.

In some preferred embodiments, the hydrolase is a glycoside hydrolase. These enzymes have a variety of uses including degradation of plant materials (*e.g.* cellulases for degrading cellulose to glucose that are used for ethanol production), food manufacturing (*e.g.* sugar inversion, maltodextrin production), and paper production (removing hemicelluloses from paper pulp).

In some preferred embodiments, the hydrolase is lipolase 100L (EC 3.1.1.3). It is used to synthesize pregabalin (marketed as by Pfizer as Lyrica®), an anticonvulsant drug used for neuropathic pain, anxiety disorders, and epilepsy. These conditions affect about 1% of the world's population. Lipolase 100L was found to reduce the required starting material by 39% and cut the waste per unit by 80%.

In some preferred embodiments, the hydrolase is a gamma-lactamase (*e.g.* EC 3.1.5.49). It is used to make Vince lactam, an intermediate for abacavir production (an antiretroviral drug for treating HIV/AIDS). It was found that changing from a stoichiometric process to a catalytic flow process reduced the number of unit operations from 17 to 12 and reduced the waste by 35%. Additionally, the use of the toxic substance cyanogen chloride is minimized.

In some preferred embodiments, the hydrolase is a Lactase (*e.g.* EC 3.2.1.108). These enzymes break apart lactose in milk into simple sugars to produce lactose-free milk. This important product serves approximately 15% of the world population that is lactose intolerant.

In some preferred embodiments, the hydrolase is a penicillin amidase (*e.g.* EC 3.5.1.11). These enzymes split penicillin into a carboxylate and 6-aminopenicillanate (6-APA). 6-APA is the core structure in natural and synthetic penicillin derivatives. These enzymes are used to produce semisynthetic penicillins tailored to fight specific infections.

In some preferred embodiments, the hydrolase is a nitralase (*e.g.* EC 3.5.5.1). These enzymes split nitriles into carboxyl groups. A nitralase is used to manufacture atorvastatin (marketed by Pfizer as Lipitor®). It catalyzes the reaction of meso-3-hydroxyglutaronitrile to ethyl (R)-4-cyano-3-hydroxybutyrate, the latter of which form the core of atorvastatin.

Hydrolases are discussed in the following references: Anastas, P.T. Handbook of Green Chemistry. Wiley-VCH-Verlag, 2009; Dunn, Peter J., Andrew Wells, and Michael T. Williams, eds. Green chemistry in the pharmaceutical industry. John Wiley & Sons, 2010.; Martinez et al., Curr. Topics Med. Chem. 13(12):1470-90 (2010); Wells et al., Organic Process Res. Dev. 16(12):1986-1993 (2012).

In some embodiments, the invention provides hydrogen peroxide producing (HPP) enzymes. In certain embodiments, the HPP enzymes are oxidases that may be of the EX 1.1.3 subgenus. In particular embodiments, the oxidase may be EC 1.1.3.3 (malate oxidase), EC 1.1.3.4 (glucose oxidase), EC 1.1.3.5 (hexose oxidase), EC 1.1.3.6 (cholesterol oxidase), EC 1.1.3.7 (aryl-alcohol oxidase), EC 1.1.3.8 (L-gulonolactone oxidase), EC 1.1.3.9 (galactose oxidase), EC 1.1.3.10 (pyranose oxidase), EC 1.1.3.11 (L-sorbose oxidase), EC 1.1.3.12 (pyridoxine 4-oxidase), EC 1.1.3.13 (alcohol oxidase), EC 1.1.3.14 (catechol oxidase), EC 1.1.3.15 (2-hydroxy acid oxidase), EC 1.1.3.16 (ecdysone oxidase), EC 1.1.3.17 (choline oxidase), EC 1.1.3.18 (secondary-alcohol oxidase), EC 1.1.3.19 (4-hydroxymandelate oxidase), EC 1.1.3.20 (long-chain alcohol oxidase), EC 1.1.3.21 (glycerol-3-phosphate oxidase), EC 1.1.3.22, EC 1.1.3.23 (thiamine oxidase), EC 1.1.3.24 (L-galactonolactone oxidase), EC 1.1.3.25 , EC 1.1.3.26, EC 1.1.3.27 (hydroxyphytanate oxidase), EC 1.1.3.28 (nucleoside oxidase), EC 1.1.3.29 (Nacylhexosamine oxidase), EC 1.1.3.30 (polyvinyl alcohol oxidase), EC 1.1.3.31, EC 1.1.3.32, EC 1.1.3.33, EC 1.1.3.34, EC 1.1.3.35, EC 1.1.3.36, EC 1.1.3.37 D-arabinono-l,4-lactone oxidase), EC 1.1.3.38 (vanillyl alcohol oxidase), EC 1.1.3.39 (nucleoside oxidase, $H_2O_2$ forming), EC 1.1.3.40 (D-mannitol oxidase), or EC 1.1.3.41 (xylitol oxidase).

Some embodiments of the invention may comprise hydroxylases. Hydroxylation is a chemical process that introduces a hydroxyl group (-OH) into an organic compound. Hydroxylation is the first step in the oxidative degradation of organic compounds in air. Hydroxylation plays a role in detoxification by converting lipophilic compounds into hydrophilic products that are more readily excreted. Some drugs (e.g. steroids) are activated or deactivated by hydroxylation. Hydroxylases are well-known in the art. Exemplary hydroxylases include proline hydroxylases, lysine hydroxylases, and tyrosine hydroxylases.

Some embodiments of the invention comprise Nitrilases (NIT). They are hydrolyzing enzymes (EC 3.5.5.1) that catalyze the hydrolysis of nitriles into chiral carboxylic acids with high enantiopurity and ammonia. NIT activity may be measured by monitoring the conversion of mandelonitirile into a (R)-mandelic acid. This results in a pH drop that may be monitored spectrophotometrically. Nitrilases are used to produce nicotinic acid, also known as vitamin B3 or niacin, from 3-cyanopyridine. Nicotinic acid is a nutritional supplement in foods and a pharmaceutical intermediate. Exemplary industrial uses are discussed in Gong et al., Microbial Cell Factories, 11(1), 142(2012)).

Some embodiments of the invention comprise hydratases. They are enzymes that catalyze the addition or removal of the elements of water. Hydratases, also known as hydrolases or hydrases, may catalyze the hydration or dehydration of C-O linkages.

Some embodiments of the invention comprise oxidoreductases. These enzymes catalyze the transfer of electrons from one molecule to another. This involves the transfer of H and O atoms or electrons from one substance to another. They typically utilize NADP or NAD+ as cofactors.

In some preferred embodiments of the invention, Oxidoreductases are used for the decomposition of pollutants such as polychlorinated biphenyls and phenolic compounds, the degradation of coal, and the enhancement of the fermentation of wood hydrolysates. The invention further includes their use in biosensors and disease diagnosis.

In some preferred embodiments, the oxidoreductase is a dehydrogenase (DHO). This group of oxidoreductases oxidizes a substrate by a reduction reaction that transfers one or more hydrides (H-) to an electron acceptor, usually NAD+/NADP+ or a flavin coenzyme such as FAD or FMN. Exemplary dehydrogenases include aldehyde dehydrogenase, acetaldehyde dehydrogenase, alcohol dehydrogenase, glutamate dehydrogenase, lactate dehydrogenase, pyruvate dehydrogenase, glucose-6-phosphate dehydrogenase, glyceraldehyde-3-phosphate dehydrogenase, sorbitol dehydrogenase, isocitrate dehydrogenase, alpha-ketoglutarate dehydrogenase, succinate dehydrogenase, and malate dehydrogenase.

In some preferred embodiments, the oxidoreductase is a ketoreductase (EC 1.1.1.184), an oxidoreductase used to make atorvastatin (marketed by Pfizer as Lipitor®). This biocatalytic process is commercially important because it substantially reduces starting materials, limits the use of organic solvents, and increases the biodegradability of the waste streams.

In some preferred embodiments, the oxidoreductase is a glucose dehydrogenase (*e.g.* EC 1.1.99.10). They are used by pharmaceutical companies to recycle cofactors used in drug production. They catalyze the transformation of glucose into gluconate. NADP+ is reduced to NADPH. This is used in Avastan production.

Cytochrome P450 (referred to as P450 or CYP) are of the E.C. 1.14 class of enzymes. (Br. J. Pharmacol. 158(Suppl 1): S215-S217 (2009)). They constitute a family of monooxygenases involved in the biotransformation of drugs, xenobiotics, alkanes, terpenes, and aromatic compounds. They also participate in the metabolism of chemical carcinogens and the biosynthesis of physiologically relevant compounds such as steroids, fatty acids, eicosanoids, fat-soluble vitamins, and bile acids. Furthermore, they are also involved in the degradation of xenobiotics in the environment such pesticides and other industrial organic contaminants. They function by incorporating one hydroxyl group into substrates found in many metabolic pathways. In this reaction, dioxygen is reduced to one hydroxyl group and one $H_2O$ molecule by the concomitant oxidation of a cofactor such as NAD(P)H.

In some preferred embodiments, the oxidoreductase is P450 (EC 1.14.14.1). It is used in the pharmaceutical industry for difficult oxidations. P450 reduces the cost, inconsistency, and inefficiency associated with natural cofactors (e.g., NADPH/NADP+). There are several advantages of using drug metabolites as active ingredients because they can show superior properties compared to the original drugs. This includes improved pharmacodynamics, improved pharmacokinetics, lower probability of drug-drug interactions, less variable pharmacokinetics and/or pharmacodynamics, improved overall safety profile and improved physicochemical properties.

In some embodiments, the P450 monooxygenase comprises a P450 sequence that is mammalian. In other embodiments, the P450 monooxygenase comprises a P450 sequence that is human. In other embodiments, the P450 monooxygenase comprises CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2U1, CYP2W1,CYP3A4, CYP3A5, CYP3A7, CYP3A43,CYP4A11, CYP4A22, CYP4B1, CYP4F2, CYP4F3, CYP4F8, CYP4F11, CYP4F12, CYP4F22, CYP4V2, CYP4X1, CYP4Z1,CYP5A1,CYP7A1, CYP7B1,CYP8A1, CYP8B1,CYP11A1, CYP11B1, CYP11B2, CYP17A1, CYP19A1, CYP20A1, CYP21A2, CYP24A1, CYP26A1, CYP26B1, CYP26C1, CYP27A1, CYP27B1, CYP27C1, CYP39A1, CYP46A1, or CYP51A1.

In some embodiments, the P450 monooxygenase comprises a P450 sequence that is of an origin selected from the group consisting of primate, mouse, rat, dog, cat, horse, cow, sheep, and goat. In other embodiments, the P450 monooxygenase comprises a P450 sequence that is of an origin selected from the group consisting of insect, fish, fungus, yeast, protozoan, and plant.

In some embodiments, the P450 is in a soluble form such as the BM3 P450 from *Bacillus megaterium. See, e.g.,* SEQ ID NO:1. In other embodiments, the BM3 P450 has one or more variant amino acids from the wild-type. In other embodiments, the P450 has at least a 90% sequence identity to SEQ ID NO:1.

In some embodiments of the invention, the cofactor is nicotinamide adenine dinucleotide + hydrogen (NADH), nicotinamide adenine dinucleotide phosphate + hydrogen (NADPH), Flavin adenine dinucleotide + hydrogen (FADH), or glutathione.

In some preferred embodiments, the oxidoreductase is a catalase such as EC 1.11.1.6. It is used in the food industry for removing hydrogen peroxide from milk prior to cheese production and for producing acidity regulators such as gluconic acid. Catalase is also used in the textile industry for removing hydrogen peroxide from fabrics.

In some preferred embodiments, the oxidoreductase is a glucose oxidase (e.g. Notatin, EC 1.1.3.4). It catalyzes the oxidation of glucose to hydrogen peroxide and D-glucono-δ-lactone. It is used, for example, to generate hydrogen peroxide as an oxidizing agent for hydrogen peroxide consuming enzymes such as peroxidase.

In some embodiments, the invention encompasses Free Radical Producing (FRP) enzymes. In some embodiments, the FRP is a peroxidase. Peroxidases are widely found in biological systems and form a subset of oxidoreductases that reduce hydrogen peroxide ($H_2O_2$) to water in order to oxidize a large variety of aromatic compounds ranging from phenol to aromatic amines. Peroxidases are very potent enzymes yet notoriously difficult to deploy in industrial settings due to strong inhibition in presence of excess peroxide. The invention provides increased reaction turnover and reduced inhibition. Thus, enzymes such as Horseradish Peroxidase (HRP) may be used at industrial scales.

Peroxidases belong to the sub-genus EC 1.11.1. In certain embodiments, the EC 1.11.1 enzyme is The EC 1.11.1 enzyme can be more specifically, for example, EC 1.11.1.1 (NADH peroxidase), EC 1.11.1.2 (NADPH peroxidase), EC 1.11.1.3 (fatty acid peroxidase), EC 1.11.1.4, EC 1.11.1.5 (cytochrome-c peroxidase), EC 1.11.1.6 (catalase), EC 1.11.1.7 (peroxidase), EC 1.11.1.8 (iodide peroxidase), EC 1.11.1.9 (glutathione peroxidase), EC 1.11.1.10 (chloride peroxidase), EC 1.11.1.11 (L-ascorbate peroxidase), EC 1.11.1.12 (phospholipid-hydroperoxide glutathione peroxidase), EC 1.11.1.13 (manganese peroxidase), EC 1.11.1.14 (diarylpropane peroxidase), or EC 1.11.1.15 (peroxiredoxin).

Horseradish peroxidase (EC 1.11.1.7) is a heme-containing oxidoreductase enzyme found in the roots of the horseradish plant *A. rusticana.* It is commonly used as a biochemical signal amplifier and tracer, as it usually acts on a chromogenic substrate together with hydrogen peroxide to produce a brightly colored product complex. It improves spectrophotometric detectability of target molecules. This characteristic of horseradish peroxidase (HRP) has been applied to permeability studies of rodent nervous system capillaries. In some embodiments of the invention, HRP is used as part of a possible remediation strategy of phenolic wastewaters due to its ability to degrade various aromatic compounds. *See* Duan et al., ChemPhysChem, 15(5), 974-980 (2014).

In other embodiments, the peroxidase may also be further specified by function, e.g., a lignin peroxidase, manganese peroxidase, or versatile peroxidase. The peroxidase may also be specified as a fungal, microbial, animal, or plant peroxidase. The peroxidase may also be specified as a class I, class II, or class III peroxidase. The peroxidase may also be specified as a myeloperoxidase (MPO), eosinophil peroxidase (EPO), lactoperoxidase (LP), thyroid peroxidase (TPO), prostaglandin H synthase (PGHS), glutathione peroxidase, haloperoxidase, catalase, cytochrome c peroxidase, horseradish peroxidase, peanut peroxidase, soybean peroxidase, turnip peroxidase, tobacco peroxidase, tomato peroxidase, barley peroxidase, or peroxidasin. In particular embodiments, the peroxidase is horseradish peroxidase.

The lactoperoxidase/glucose oxidase (LP/GOX) antimicrobial system occurs naturally in bodily fluids such as milk, saliva, tears, and mucous (Bosch et al., J.Applied Microbiol., 89(2), 215-24 (2000)). This system utilizes thiocyanate (SCN-) and iodide (I-), two naturally occurring compounds that are harmless to mammals and higher organisms (Welk et al. Archives of Oral Biology, 2587 (2011)). LP catalyzes the oxidation of thiocyanate and iodide ions into hypothiocyanite (OSCN-) and hypoiodite (OI-), respectively, in the presence of hydrogen peroxide ($H_2O_2$). The $H_2O_2$ in this system is provided by the activity of GOX on β-D-glucose in the presence of oxygen. These free radical compounds, in turn, oxidize sulfhydryl groups in the cell membranes of microbes (Purdy, Tenovuo et al. Infection and Immunity, 39(3), 1187 (1983); Bosch et al., J.Applied Microbiol., 89(2), 215-24 (2000), leading to impairment of membrane permeability (Wan, Wang et al. Biochemistry Journal, 362, 355-362 (2001)) and ultimately microbial cell death.

Some embodiments of the invention comprise transferases. "Transferase" refers to a class of enzymes that transfer specific functional groups from one molecule to another. Examples of groups transferred include methyl groups and glycosyl groups. Transferases are used for treating substances such as chemical carcinogens and environmental pollutants. Additionally, they are used to fight or neutralize toxic chemicals and metabolites found in the human body.

In some preferred embodiments, the transferase is a transaminase. A transaminase or an aminotransferase catalyzes a reaction between an amino acid and an α-keto acid. They are important in the synthesis of amino acids. In transamination, the $NH_2$ group on one molecule is exchanged with the =O from another group (*e.g.* a keto group) on the other molecule.

In more preferred embodiments, the transaminase is ω-transaminases (EC 2.6.1.18). It is used, among other things, to synthesize sitagliptin (marketed by Merck and Co. as Januvia®, an antidiabetic drug). Engineered ω-transaminases were found to improve biocatalytic activity by, for example, 25,000 fold, resulting in a 13% overall increase in sitagliptin yield and 19% reduction in overall process waste.

Due to their high stereoselectivity for substrates and stereospecificity for products, ω-transaminases can be utilized to make unnatural amino acids and optically pure chiral amines or keto acids (Mathew & Yun, ACS Catalysis 2(6), 993-1001 (2012)). ω-Transaminases also have applications in biocatalytic chiral resolution of active pharmaceutical intermediates, simplifying the process over conventional chemical methods. (Schätzle et al., Anal. Chem. 81(19):8244-48 (2009).)

In some preferred embodiments, the transferase is a thymidylate synthetase *(e.g.* EC 2.1.1.45). These enzymes are used for manufacturing sugar nucleotides and oligosaccharides. They catalyze, for example, the following reaction:

5,10-methylenetetrahydrofolate + dUMP ⇌ dihydrofolate + dTMP.

In some preferred embodiments, the transferase is a glutathione S-transferase *(e.g.* EC 2.5.1.18). These enzymes catalyze glutathione into other tripeptides. They are used in the food industry as oxidizing agents as well as in the pharmaceutical industry to make anti-aging drugs and skin formulations.

In some preferred embodiments, the transferase is a glucokinase (e.g. EC 2.7.1.2). These enzymes facilitate the phosphorylation of glucose to glucose-6-phosphate. They are used in the food industry to reduce the glucose concentration in their production streams and as in the pharmaceutical industry to make diabetes drugs.

In some preferred embodiments, the transferase is a riboflavin kinase (e.g. EC 2.7.1.26). In a more preferred embodiment, a riboflavin kinase is used to produce flavin mononucleotide (FMN) in the food industry. FMN is an orange-red food color additive and an agent that breaks down excess riboflavin (vitamin $B_2$). Riboflavin kinase catalyzes, for example, the following reaction:

ATP + riboflavin ⇌ ADP + Flavin mononucleotide (FMN).

Some embodiments of the invention comprise ene reductases (EREDS). These enzymes catalyze alkene reduction in an NAD(P)H-dependent manner. Examples of ene reductases include The FMN-containing Old Yellow Enzyme (OYE) family of oxidoreductases (EC 1.6.99), clostridial enoate reductases (EnoRs, C 1.3.1.31), flavin-independent medium chain dehydrogenase/reductases (MDR; EC 1.3.1), short chain dehydrogenase/reductases (SDR; EC 1.1.1.207-8), leukotriene B4 dehydrogenase (LTD), quinone (QOR), progesterone 5b-reductase, rat pulegone reductase (PGR), tobacco double bond reductase (NtDBR), *Cyanobacterial* OYEs, LacER from *Lactobacillus casei,* Achr-OYE4 from *Achromobacter* sp. JA81, and Yeast OYEs.

Some embodiments of the invention comprise imine reductases (IREDS). Imine reductases (IRED) catalyze the synthesis of optically pure secondary cyclic amines. They may convert a ketone or aldehyde substrate and a primary or secondary amine substrate to form a secondary or tertiary amine product compound. Exemplary IREDs are those from *Paenibacillus elgii* B69, *Streptomyces ipomoeae* 91-03, *Pseudomonas putida* KT2440, and *Acetobacterium woodii.* IREDs are discussed in detail in Int'l Pub. No. WO2013170050.

In some embodiments of the invention, the enzymes are lyases. They catalyze elimination reactions in which a group of atoms is removed from a substrate by a process other than hydrolysis or oxidation. A new double bond or ring structure often results. Seven subclasses of lyases exist. In preferred embodiments, pectin lyase is used to degrade highly esterified pectins *(e.g.* in fruits) into small molecules. Other preferred embodiments of the invention comprise oxynitrilases (also referred to as mandelonitrile lyase or aliphatic (R)-hydroxynitrile lyase). They cleave mandelonitrile into hydrogen cyanide + benzaldehyde.

In a preferred embodiment, the lyase is a hydroxynitrile lyase *(e.g.* EC 4.1.2, a mutation of a *Prunus amygdalus* lyase). Hydroxynitrile lyases catalyze the formation of cyanohydrins which can serve as versatile building blocks for a broad range of chemical and enzymatic reactions. They are used to improve enzyme throughput and stability at a lower pH and is used for producing clopidogrel (Plavix®). The reaction process is described in Glieder et al., Chem. Int. Ed. 42:4815 (2003).

In another preferred embodiment, the lyase is 2-deoxy-D-ribose phosphate aldolase (DERA, EC 4.1.2.4). It is used for forming statin side chains, e.g. in Lipitor production.

In another preferred embodiment, the lyase is (R)-mandelonitrile lyase (HNL, EC 4.1.2.10). It is used to synthesize *Threo*-3-Aryl-2,3-dihydroxypropanoic acid, a precursor cyanohydrin used to produce Diltiazem. Diltiazem is a cardiac drug that treats high blood pressure and chest pain (angina). Lowering blood pressure reduces the risk of strokes and heart attacks. It is a calcium channel blocker. Ditiazem and its production are described in Dadashipour and Asano, ACS Catal. 1:1121-49 (2011) and Aehle W. 2008. Enzymes in Industry, Weiley-VCH Verlag, GmbH Weinheim.

In another preferred embodiment, the lyase is nitrile hydratase (EC 4.2.1). It is used commercially to convert 3-cyanopyridine to nicotinamide (vitamin B3, niacinamide). It is also used in the preparation of levetiracetam, the active pharmaceutical ingredient in Keppra®.

In another preferred embodiment, the lyase is a Phenyl Phosphate Carboxylase. They are used, *e.g.,* for phosphorylating phenol at room temperature and under sub-atmospheric $CO_2$ pressure. These enzymes catalyze the synthesis of 4-OH benzoic acid from phenol and $CO_2$ with 100% selectivity. 4-OH benzoic acid is used in the preparation of its esters. In more preferred embodiments, the enzymes are used for producing parabens that are used as preservatives in cosmetics and opthalmic solutions.

In some embodiments of the invention, the enzyme is a carbonic anhydrase *(e.g.* EC 4.2.1.1). Carbonic anhydrases are ubiquitous metalloenzymes present in every organism. They are among the most efficient enzymes known and serve multiple physiological roles including $CO_2$ exchange, pH regulation, and $HCO_3^-$ secretion. Carbonic anhydrase also has potential industrial applications in $CO_2$ sequestration and calcite production. *See* Lindskog & Silverman, (2000), The catalytic mechanism of mammalian carbonic anhydrases EXS 90:175-195 (W. R. Chegwidden et al. eds. 2000); In The Carbonic Anhydrases: New Horizons 7th Edition pp. 175-95 (W. R. Chegwidden et al. eds. 2000); McCall et al., J. Nutrition 130:1455-1458 (2000); Boone et al., Int'l J. Chem. Engineering Volume 2013: 22-27 (2013).

In some embodiments of the invention, the enzyme is an isomerase. Isomerases catalyze molecular isomerizations, *i.e.* reactions that convert one isomer to another. They can facilitate intramolecular rearrangements in which bonds are broken and formed or they can catalyze conformational changes. Isomerases are well known in the art.

In preferred embodiments, isomerases are used in sugar manufacturing. In more preferred embodiments, the isomerase is Glucose isomerase, EC 5.3.1.18. In other embodiments, the glucose isomerase is produced by *Actinoplanes missouriensis, Bacillus coagulans* or a *Streptomyces* species. Glucose isomerase converts D-xylose and D-glucose to D-xylulose and D-fructose, important reactions in the production of high-fructose corn syrup and in the biofuels sector.

In another preferred embodiment, the isomerase is Maleate cis-trans isomerase (EC 5.2.1.1). It catalyzes the conversion of maleic acid into fumaric acid. Fumaric acid is important for the biocatalytic production of L-aspartic acid, L-malic acid, polyester resins, food and beverage additives, and mordant for dyes.

In another preferred embodiment, the isomerase is linoleate cis-trans isomerase (EC 5.2.1.5). It catalyzes the isomerization of conjugated linoleic acid (CLA). CLA has been reported to have numerous potential health benefits for treating obesity, diabetes, cancer, inflammation, and artherogenesis. Different isomers of CLA may exert differential physiological effects. Thus, the enzyme is used to prepare single isomers.

In another preferred embodiment of the invention, the isomerase is triosephosphate isomerase (EC 5.3.1.1). It catalyzes the interconversion of D-glyceraldehyde 3-phosphate and dihydroxyacetone phosphate. In combination with transketolases or aldolases, triosephosphate isomerase is used in the stereoselective multienzyme synthesis of various sugars or sugar analogs. A preferred embodiment is the one-pot enzymatic preparation of D-xylulose 5-phosphate. This synthesis starts with the retro-aldol cleavage of fructose 1,6-biphosphate by D-fructose 1,6-biphosphate aldolase (EC 4.1.2.13). The following racemization, triosephosphate isomerase facilitates the generation of two equivalents of D-glyceraldehyde 3-phosphate that is converted into xylulose 5-phosphate by transketolase (EC 2.2.1.1)

In other embodiments of the invention, the enzyme is a Ligase. These enzymes catalyze the formation of covalent bonds joining two molecules together, coupled with the hydrolysis of a nucleoside-triphosphate. Ligases are well-known in the art and are commonly used for recombinant nucleic acid applications. In a preferred embodiment, the DNA ligase is EC 6.5.1.1.

In a preferred embodiment, the ligase is Acetyl-CoA Carboxylase (EC 6.4.1.2, ACC). ACC has a role at the junction of the lipid synthesis and oxidation pathways. It is used with the inventions disclosed herein for clinical purposes such as the production of antibiotics, diabetes therapies, obesity, and other manifestations of metabolic syndrome.

In another preferred embodiment, the ligase is Propionyl-CoA Carboxylase (PCC, EC 6.4.1.3). It catalyzes the biotin-dependent carboxylation of propionyl-CoA to produce D-methylmalonyl-CoA in the mitochondrial matrix. Methylmalyl-CoA is an important intermediate in the biosynthesis of many organic compounds as well as the process of carbon assimilation.

In some embodiments, the methods described herein use recombinant cells that express the enzymes used in the invention. Recombinant DNA technology is known in the art. In some embodiments, cells are transformed with expression vectors such as plasmids that express the enzymes. In other embodiments, the vectors have one or more genetic signals, e.g., for transcriptional initiation, transcriptional termination, translational initiation and translational termination. Here, nucleic acids encoding the enzymes may be cloned in a vector so that they are expressed when properly transformed into a suitable host organism. Suitable host cells may be derived from bacteria, fungi, plants, or animals as is well-known in the art.

Although BNCs (Level 1) provide the bulk of enzyme immobilization capability, they are sometimes too small to be easily captured by standard-strength magnets. Thus, sub-micrometric magnetic materials (Level 2) are used to provide bulk magnetization and added stability to Level 1. Commercially available free magnetite powder, with particle sizes ranging from 50-500 nm, is highly hydrophilic and tends to stick to plastic and metallic surfaces, which, over time, reduces the effective amount of enzyme in a given reactor system. In addition, powdered magnetite is extremely dense, thus driving up shipping costs. It is also rather expensive - especially at particle sizes finer than 100 nm. To overcome these limitations, low-density hybrid materials consisting of magnetite, non-water-soluble cross-linked polymers such as poly(vinylalcohol) (PVA) and carboxymethylcellulose (CMC), have been developed. These materials are formed by freeze-casting and freeze-drying water-soluble polymers followed by cross-linking. These materials have reduced adhesion to external surfaces, require less magnetite, and achieve Level 1 capture that is at least comparable to that of pure magnetite powder.

In one embodiment, the continuous macroporous scaffold has a cross-linked polymeric composition. The polymeric composition can be any of the solid organic, inorganic, or hybrid organic-inorganic polymer compositions known in the art, and may be synthetic or a biopolymer that acts as a binder. Preferably, the polymeric macroporous scaffold does not dissolve or degrade in water or other medium in which the hierarchical catalyst is intended to be used. Some examples of synthetic organic polymers include the vinyl addition polymers (e.g., polyethylene, polypropylene, polystyrene, polyacrylic acid or polyacrylate salt, polymethacrylic acid or polymethacrylate salt, poly(methylmethacrylate), polyvinyl acetate, polyvinyl alcohol, and the like), fluoropolymers (e.g., polyvinylfluoride, polyvinylidenefluoride, polytetrafluoroethylene, and the like), the epoxides (e.g., phenolic resins, resorcinol - formaldehyde resins), the polyamides, the polyurethanes, the polyesters, the polyimides, the polybenzimidazoles, and copolymers thereof. Some examples of biopolymers include the polysaccharides (e.g., cellulose, hemicellulose, xylan, chitosan, inulin, dextran, agarose, and alginic acid), polylactic acid, and polyglycolic acid. In the particular case of cellulose, the cellulose may be microbial- or algae-derived cellulose. Some examples of inorganic or hybrid organic-inorganic polymers include the polysiloxanes (e.g., as prepared by sol gel synthesis, such as polydimethylsiloxane) and polyphosphazenes. In some embodiments, any one or more classes or specific types of polymer compositions provided above are excluded as macroporous scaffolds.

In order that the invention described herein may be more fully understood, the following examples are set forth. It should be understood that these examples are for illustrative purposes only and are not to be construed as limiting this invention in any manner.

**Example 1** - **Thermoplastics and Magnetite Blends for 3D Printing by Melting and Extrusion**

Materials: Polyamide 12 (Nylon 12, Cat No. AdSint PA12) and polystyrene (Cat. No. Coathylene PS Sint) were purchased from Advanc3D Materials GmbH (Hamburg, Germany). Polypropylene (Cat. No. Polyaxis PD 2000) sample was obtained from A. Schulman (Fairlawn, Ohio). Iron oxide (Cat. No. QM-D50/4 Magnetite) with a particle size smaller than 10 $\mu$m was obtained from Reade Advanced Materials (East Providence, RI) and iron (II, III) oxide particle size smaller than 5 $\mu$m from Sigma Aldrich (Cat. No. 310069-500G, St Louis, MO). The blowing agents used were sodium bicarbonate (Sigma Aldrich, Cat. No. SX0320-1) and citric acid (VWR, Radnor, Pennsylvania, Cat. No. BDH8003-500G). Dimethyl sulfoxide from Fisher Scientific (DMSO, Cat. No. D128-4, Hampton, NH) was used without further purification.

Extrusion blending of thermoplastics and magnetite: Different proportions of polymers, magnetite, and blowing agent powders were placed in a glass vessel and mixed in a rotary tumbler (Lortone, Inc. Model 3A) for several minutes. The powder mixtures were fed into a single screw Filabot extruder (Cat. No. EX00334) and further blended this way at different ranges of temperature and speeds depending upon the polymer matrix composition and blowing agent combination. The size of the composite magnetic materials obtained from the extrusion process was reduced in a stainless-steel blender (Waring Commercial Cat. No. CB15T) to a size of 2.82 mm and smaller by grinding them at high speed at -78.5°C for several minutes various times. Then the material particle size was further reduced to 60 microns and smaller by cooling it at -184.44°C and feeding the particles to an impact mill (Vortec Model M-1) operating at 20,000 RPM, this process was repeated as many times as required to obtain that particle size.

Extruded composite example 1: A blend of 100 g of polypropylene (46% w/w), magnetite (46% w/w), and sodium bicarbonate (8% w/w) powders were mixed in a glass cylinder for 15 minutes in the rotary tumbler. The solid blended powders were fed to the extruder and mixed at 175°C and 21 RPM.

Extruded composite example 2: Another blend was made using 100 g of a powder mixture of polystyrene (48% w/w), magnetite (<10 um, 48% w/w) and sodium bicarbonate (4% w/w). The powders were pre-mixed in the rotary tumbler for 15 min. The mixture was further blended in the extruder at the temperature range of 170° to 175°C and a speed of 12 RPM.

Thermoplastics and magnetite blends for 3D printing by dissolution coating: powders of nylon 12 and magnetite (<5 $\mu$m) 50/50 % w/w were added into a glass vessel containing DMSO in a ratio of 90% v/v of solvent to 10% v/v of powder materials. The mixture was homogeneously stirred at 500 RPM and the temperature was gradually raised to 140°C to allow nylon 12 to dissolve. The material was kept in solution for 15 min. After that time, the mixture of dissolved nylon 12 and dispersed magnetite was allowed to slowly cool down while stirring at 500 RPM. In this fashion, magnetite particles were incorporated into the nylon 12 particles that started to precipitate out of DMSO solution during the cooling down process. The magnetite nylon 12 composite particles were purified using a neodymium magnet to remove as much of the DMSO as possible. Then the composite powder was rinsed with cold water several times, after that the material was dried in a vacuum oven at 100°C for 24 hours to remove the remaining absorbed DMSO. Finally, the material was sieved using a 100 $\mu$m opening mesh.

The microstructural features of the developed composite materials before and after sintering were observed under a field emission scanning electron microscope (FESEM, Tescan Mira3) after being sputter coated (7 nm in thickness) with a gold-palladium target.

The morphology of the extruded polypropylene magnetite composite after cryogenic grinding, sintering, and enzyme immobilization was evaluated by SEM are shown in Figures 1A-1C, respectively. The shape of the cryogenically grinded composite particles is not perfectly round but is rather regular. The particle size distribution of the polypropylene magnetite composite after cryogenic grinding is the range of 1.945 to 248.9 microns (Figure 3). Particles smaller than 37 microns were below 35% of the total and 60% were below 62.23 microns. For the sintering process, the polypropylene magnetite composite powder was screened with a 100 micron opening mesh. This is consistent with the particles observed in the SEM image (Figure 1B). The particles below 40 microns were not removed. This maximized the utilization of the cryo-ground composite powders to a 75% of the total. The flowing ability of the powders was good enough for sintering. Figure 1B displays a successful sintering process of the polypropylene magnetite extruded composite powders that holds the characteristic solidification, coarsening, and porosity features of this process due to the fusing of the powders. Furthermore, it is possible to see residual sodium bicarbonate particles with a needle like shape (approximately 0.5 microns in diameter and 8 microns in length). The sodium bicarbonate residual material was easily removed with a water rinse before enzyme immobilization, also shown by SEM. Figure 1C (500 nm scale bar) shows the attachment of faceted magnetite nanoparticles (MNP) in a clusters form (level 1) onto the surface of the sintered polypropylene magnetite composite (level 2) after invertase immobilization. This was not previously observed on the sintered material. The density of the MNP is high on the surface of the sintered material. This correlates with the enzymatic immobilization and activity yield results.

Figures 2A and 2B show the morphology of the nylon 12 magnetite composite produced from the dissolution coating process. The backscatter SEM image of the material (Figure 2A) with a 50 micron scale bar shows the magnetite particles with a lighter color. This is due to the higher atomic number of magnetite densely embedded on the surface of nylon 12 powder (dark color particles). The size of the magnetite coated nylon 12 powders was approximately 20 to 25 microns in size when measuring 150 particles from SEM images. The assembly of the composite is further shown in Figure 2B (5 microns scale bar) as blossom-like or dendritic high surface area nylon 12 particles highly coated with faceted magnetite particles.

**Example 2** - **Thermoplastic-Magnetite Blend Sintering and 3D printing**

3D printing of the thermoplastic-magnetite composite was conducted with a Sintratec© Kit (https://sintratec.com/, Switzerland). Objects were computer modeled with Autodesk Inventor (https://www.autodesk.com/). Polyamide 12 (Nylon 12, AdSint PA12) was purchased from Advanc3D Materials GmbH. Polypropylene (Polyaxis, Fairlawn, Ohio, Cat. No. PD 2000) sample was obtained from A. Schulman. Iron oxide (Cat. No. QM-D50/4 Magnetite) with a particle size smaller than 10 $\mu$m was obtained from Reade Advanced Materials and iron (II, III) oxide particle size smaller than 5 $\mu$m from Sigma Aldrich (Cat. No.310069-500G). The blowing agent used was sodium bicarbonate (EMD Millipore, Cat. NO. SX0320-1). Two types of low-density fumed silica were obtained from Cabot (CAB-O-SIL TS-530, CAB-O-SIL TS-610). An additional type of silica was obtained from Evonik (AEROSIL R 812). A number of properties were optimized, particularly high young's moduli, resolution, void fraction, yield strength, and magnetic moment with low elastic ranges and hydrophobicity. Printing parameters (powder flowability, chamber temperature, print bed temperature and laser speed) were investigated and ensure maximum surface area while maintaining object strength.

Powder Flowability - Flowability testing was conducted using a plate with etched rectangular channels and a flat leveler. Powder was poured onto the plate and the leveler was scraped across the surface of the channel. If the powder was unable to spread evenly, low density fumed silica was incorporated to the composition to improve flowability. Extruded composite blends of polypropylene (46% w/w), magnetite (<10 $\mu$m 46% w/w), and sodium bicarbonate (8% w/w) did not require silica for printing but after adding silica (R 812 0.25% w/w) the sample became noticeably more fluid. A physical blend of nylon 12 (49.625% w/w), magnetite (<5 $\mu$m 49.625% w/w), BC (0.5% w/w), and silica (TS-610 0.25% w/w) was able to sinter in contrast with its counterpart without added silica. All three silicas were analyzed and similar results were observed. Composites powders with processed base powders and added filler thermoplastics often required additional silica to achieve desired flowability. A range of 0.25-0.75% (w/w) was required to produce spreadable powders. Compositions that did not require the addition of flowing agents, however, were more desirable for the final process.

Chamber Temperature - The chamber temperature refers to the ambient temperature in the printer interior. Before printing, the chamber is heated until the air, powder, and structural components are at a uniform temperature to minimize the effects of heat transfer during printing. Heat transfer between sintered layers and fresh powder can cause the sintered piece to cause print failure. In general, the best chamber temperatures were found within 10°C of the glass transition temperature. For nylon 12-magnetite composites, the optimal print chamber temperature was 140°C. The chamber temperature for polypropylene-magnetite composites was iterated from 110-130°C with the optimal chamber temperature being 120°C.

Fabrication Surface Temperature - The fabrication surface temperature is the temperature of the surface of the print bed. The surface temperature is controlled by an infrared temperature sensor and an array of three heat lamps. This parameter is responsible for bringing the surface temperature right below the glass transition temperature. In general, optimal surface temperatures were found within 5°C of the glass transition temperature. For nylon 12-magnetite composites, optimal print surface temperatures were between 160° and 170°C depending on the composition. Blends of nylon 12 (49.875% w/w), 49.875% [BC 2% w/w, magnetite <10 $\mu$m 98% w/w], and silica (TS-610 0.25% w/w) were found to sinter best with a surface temperature of 164°C while 79.75% [46% nylon 12, 46% <10 $\mu$m magnetite, 8% sodium bicarbonate] (w/w), nylon 12 (20% w/w), and silica (TS-610 0.25% w/w) were best sintered with a surface temperature of 170°C. The surface temperature for polypropylene-magnetite composites was iterated from 110-155°C, with the optimal surface temperature of 130°C.

Laser Scan Speed - This is the speed of laser travel during sintering. This parameter modifies adhesion between layers, pore sizes, material strength, and sintering ability. The laser scan speed is modified to ensure that powders bond together on each layer due to the varying levels to which the powders will absorb the energy from the laser. Laser speed also affects sintering of different samples due to their material composition and packing structure. In some cases, the thermoplastic is not as readily able to interface with itself (*i.e.,* different coatings or concentrations). Thus, it needs a longer exposure time to allow some powder to reach melting temperature and become amorphized to reach sintered counterparts. For this same reason, scan speed affects the pore sizes. The laser speed was varied from 100 to 950 mm/s. Depending on the compositions, laser speeds were adjusted to flash the powders just above the glass transition temperature. For polypropylene and nylon samples, the optimal laser speed was found to be 650 mm/s.

Varying the concentration of polypropylene, magnetite, sodium bicarbonate, and the conditions under which the blends were made led to different optimization parameters. The following three example prints were successfully sintered, developed strong stable prints with rigidity, and the ability to withstand functionalization with BNCs and enzymatic reactions:

a. Extruded composite blend of polypropylene (46% w/w), magnetite (<10 $\mu$m 46% w/w), and sodium bicarbonate (8% w/w) at chamber temperature of 110°C, surface temperature of 125°C, laser speed of 650 mm/s
b. Extruded composite blend of polypropylene (48% w/w), magnetite (<10 $\mu$m 48% w/w), and sodium bicarbonate (4% w/w) at chamber temperature of 110°C, surface temperature of 125°C, laser speed of 350 mm/s
c. Extruded composite blend of polypropylene (36% w/w), magnetite (<10 $\mu$m 56% w/w), and sodium bicarbonate (8% w/w) at chamber temperature of 119°C, surface temperature of 129°C, laser speed of 350 mm/s

The prints vary in microscopic porosity due to not only these print conditions but their material properties. The optimization of print parameters aimed at maximizing functionalization with BNCs and enzymatic reactions.

Varying the concentration of nylon, BC, silica, magnetite, and sodium bicarbonate and the conditions under which the blends were made led to different optimization parameters. The following three example prints were successful in sintering, developing strong stable prints with rigidity, and the ability to withstand functionalization with BNCs and enzymatic reactions:

a. Physical blend of nylon 12 (49.875% w/w), 49.875% [2% BC, 98% magnetite <10 $\mu$m] (w/w), and silica (TS-610 0.25% w/w) at chamber temperature of 140°C, surface temperature of 164°C, laser speed of 300mm/s
b. Physical blend of 99.75% [Dissolution coating of nylon 12 (50% w/w), 50% [BC (2% w/w), magnetite (<10 $\mu$m 98% ww)]], silica (TS-610 0.25% w/w) at a chamber temperature of 140°C, surface temperature of 160°C, laser speed of 200 mm/s
c. Physical blend of 79.75% [extruded composite blend of magnetite (<10 $\mu$m 46% w/w), nylon 12 (46% w/w), and sodium bicarbonate (8% w/w)], nylon 12 (20% w/w), silica (TS-610 0.25% w/w) at chamber temperature of 140°C, surface temperature of 170°C, laser speed of 300 mm/s.

The prints varied in microscopic porosity due to not only sintering conditions but also material properties such as powder size. The optimization of print parameters aimed at maximizing functionalization with BNCs and enzymatic reactions.

Printing of polypropylene-magnetite objects: Powders of an extruded composite blend of magnetite (<10 $\mu$m 46% w/w), polypropylene (46% w/w), and sodium bicarbonate (8% w/w) were prepared as previously described. The powders were placed in the print and reservoir beds of the Sintratec Kit and preheated at 115°C for one hour. Strips, beads, crosses, and mixing elements were sintered at a 115°C chamber temperature, 125°C surface temperature, 650 mm/s laser scan speed, and 100 $\mu$m layer height. Once sintering was complete, the printed parts were left in the chamber to slowly cool for one hour. For single layer strips, the length was 30 mm, the width was 3 mm, the thickness was 0.1 mm, and the mass was 0.026 g, the theoretical surface area was 262 mm$^2$. For beads, the diameter was 3 mm, the mass was 0.01 g, the volume was 0.014 cm$^2$, the theoretical surface area was 0.283 cm$^2$. For crosses, the length was 8mm, the width was 2 mm, the thickness was 5 mm, the mass was 0.11 g, the volume was 0.147 cm$^3$, density of 0.75 grams per cm$^3$, the theoretical surface area was 2.16 cm$^2$. For the static mixing element, the length was 12.7 mm, the diameter was 12.7 mm, the mass was 0.35 grams, the volume was 0.46 cm$^3$, and the density was 0.76 grams per cm$^3$. Figures 4A-4D show 3D models and Figures 4E-4H show high-resolution images of the 3D object samples after printing.

An estimation of the surface area created during the sintering process of the polypropylene magnetite composite was done using the following rationale. The values are measured by mercury porosimetry and BET Brunauer-Emmett-Teller (BET) surface characterization. The theoretical density of the composite was calculated using equation 1 (Sharma, J. Materials Engin. and Performance 12:324-330 (2003)):

$$\rho_{composite}=m_C/v_C =(m_P+m_M)/(v_P+v_M ) \text{ (eq. 1)}$$

Where mc is the composite mass, vc the composite volume, $m_P$ is the mass reinforcing particles, $m_M$ the mass of the polymer matrix, $v_P$ the volume of reinforcing particles and $v_M$ the polymer matrix volume. Using the density of polypropylene as 0.92 g/cm$_3$ and 5 g/cm$_3$ for magnetite in a 50 to 50 polypropylene to magnetite weight ratio, the respective volumes of polypropylene and magnetite relative to 2 grams of material are 1.087 cm$^3$ and 0.2 cm$^3$. Therefore, the density of the composite is 1.55g/cm$^3$. The theoretical porosity percentage of the 3D printed sintered materials can be calculated by using the previously calculated composite density ($\rho$composite) and the density of the actual sintered materials ($\rho$sm) as follow (equation 2):

$$\%Porosity = 100x[1-(\rho_{sm}/\rho_{composite})] \text{ (eq. 2)}$$

The sintered material density was determined as 0.75 g/cm$_3$ by dividing the mass of the sintered object by the actual solid volume of the design (calculated by the CAD software). Hence the theoretical porosity of the sintered materials was 52%. This way, the volume occupied by the actual composite particles was estimated by multiplying the solid fraction (0.48) by the volume of the sintered material. Then, assuming a spherical shape of PP-Mag particles and an average diameter of 34 microns, calculate the particle size and its respective percentage in the utilized size range. The amount of particles used to create that volume was calculated by dividing the solid volume of the sintered object by the volume of a single particle. Then the obtained particles were reduced to two thirds of the total, assuming one third of them lost their shape by solidification during the sintering process. Multiplying the surface area of one single particle (SA=3V/r, $3.64 \times 10^{-4}$ cm$^2$) by the total amount of available particles in the sintered 3D object provided the theoretical expected surface area. This was expected to be higher since the surface area created by the porosity and surface roughness was not considered in this approximation. The average specific surface area was calculated as 762 cm$^2$ per gram of sintered material.

It was demonstrated that 3D magnetic objects were able to be printed (Figures 4E-4H) with the powder composition of magnetite blended with thermoplastics so that they maintained rigidity and porosity for the immobilization of enzymes. Objects were used to immobilize BNCs.

The printed geometries (Figures 4A-4D) were designed for different applications. Strips were designed and engineered for quick optimization of the immobilization of enzymes. Beads were designed and engineered for the immobilization of BNCs and to fit comfortably in 96-well microplates for the screening of enzyme activities. Their shape allows for easy removal from the wells with external magnets. Crosses were designed and engineered for the immobilization of BNCs in small and medium batch reactors. Crosses can also be used as stir elements when combined with stir plates. Static mixers were designed and engineered for immobilization of BNCs in continuous flow systems. Static mixers consist of individual mixing elements placed in series. Adjacent elements are rotated 90° with respect to each other. Pipe housing encloses the static mixer and provides connections within continuous flow systems.

**Example 3** - **Immobilization of Bionanocatalysts (BNCs) on 3D Printed Scaffolds**

**Invertase Immobilization on Sintered Materials**: Invertase from baker's yeast was purchased from Sigma Aldrich (St. Louis, MO, USA, Cat. No. I4504). All water was obtained from a BamStead Nanopure water purifier (Thermo Scientific, 18.2 MOhm-cm). Iron oxide nanoparticles (NPs) were prepared as previously described (U.S. Patent No. 9,597,672; Int'l Pub. No. WO2018102319) and stored under a N$_2$ sparged atmosphere at pH 11 at 4°C.

Nanoparticles were ultrasonicated (Fisher Scientific) on the day of immobilization for 1 minute at 40% amplitude. NP solutions of 1000 $\mu$g/mL were prepared from a 24 mg/mL stock solution with pH 3 water. Cold NP solution was added rapidly to cold enzyme solution containing: 200 $\mu$g/mL of invertase in pH 7 water. The NP-enzyme mix was then added to a centrifuge tube (Eppendorf) and incubated for 1 hour at 25°C. The immobilization yield was quantified by the Bradford method (Bradford reagent: Quick Start™ Bradford 1x Dye Reagent #5000205 from BioRad) by comparing against invertase enzyme standards.

A reducing sugar substrate assay was developed to assess the activity of free and immobilized invertase. The assay measured the hydrolysis of raffinose into fructose and melibiose using 4-hydroxybenzhydrazide (PAHBAH) purchased from Sigma Aldrich (cat. H9882). D-(+)-Raffinose pentahydrate (Cat No. R0250) was purchased from Fisher Scientific (Hampton, NH, USA, Cat. No. AAA1831309) and D-(+)-glucose was purchased from EMD Millipore (Cat. No.DX0145-3). 10X PBS buffer was purchased from Thermo Scientific (Waltham, MA, USA, Cat. SH30258.02).

A reaction mix was prepared with 1 mM raffinose in 1X PBS buffer (pH 7.4) and pH 7 water. Reaction mix was added to the immobilized and free enzyme systems, then incubated for 5 minutes at 25°C. The resulting supernatant was collected and added to a 5 mg/mL solution of PAHBAH in 0.5 M NaOH (cat. 7708-10) purchased from Macron Fine Chemicals (Center Valley, PA, USA). The supernatant-PAHBAH mix was then boiled for 5 minutes and quantified via absorbance at 410 nm and compared to a standard curve of glucose.

The immobilization yields of invertase on the 3D printed cross and bead were 75% and 25%, respectively (Figure 5A). The relative activities of immobilized invertase for the hydrolysis of raffinose by the cross and bead were 146% and 50%, respectively, when normalized by the immobilization yield (Figure 5B).

It was demonstrated that invertase immobilized on the 3D printed cross had comparable activity to free invertase. When normalized, the invertase immobilized on the cross demonstrated a 1.5-fold increase in activity for the hydrolysis of raffinose compared to free invertase.

**CALB Immobilization on Sintered Materials:** Lipase B from *Candida antarctica* (CALB) was purchased from Sigma Aldrich (Cat. No. 62288). All water was obtained from a BamStead Nanopure water purifier (Thermo Scientific, 18.2 MOhm-cm). Iron oxide nanoparticles (NPs) were prepared as previously described (U.S. Patent No. 9,597,672; Int'l Pub. No. WO2018102319) and stored under a $N_2$ sparged atmosphere at pH 11 at 4°C.

Nanoparticles were ultrasonicated (Fisher Scientific) on the day of immobilization for 1 min at 40% amplitude. NP solutions of 500 $\mu$g/mL were prepared from a 24 mg/mL stock solution with pH 3 water. Cold NP solution was added rapidly to cold enzyme solution containing: 200 $\mu$g/mL of CALB in pH 7 water. The NP-enzyme mix was then added to a centrifuge tube (Eppendorf) and incubated for 2 hours at 25°C. The immobilization yield was quantified by the Bradford method (Bradford reagent: Quick Start™ Bradford 1x Dye Reagent #5000205 from BioRad) by comparing against CALB enzyme standards.

To assess the activity of immobilized and free CALB, a colorimetric assay of glycerol ester hydrolysis was developed. The assay measured the hydrolysis of para-nitrophenyl laurate (p-NPL) into para-nitrophenol (p-NP) and lauric acid. The p-NPL was purchased from Fisher Scientific (cat. 50-014-39022) and p-NP was purchased from Sigma Aldrich (cat. 48549).

A reaction mixture was prepared with 100 $\mu$M p-NPL in 50 mM Tris (pH 7.5) (cat. RGF-3350) purchased from KD Medical (Columbia, MD, USA). The reaction mixture was added to the immobilized and free enzyme systems, then incubated for 4 minutes at 25°C. The supernatant was collected, then p-NP was quantified by measuring absorbance at 400 nm and compared to a standard of p-NP.

The immobilization yields of CALB on the 3D printed cross and bead (in triplicate) were 99% $\pm$ 2% and 100%, respectively (Figure 6A). The relative activities of immobilized CALB for the hydrolysis of p-NPL by the cross and bead were 186% $\pm$ 16% and 18% $\pm$ 1%, respectively, when normalized by the immobilization yield. (Figure 6B).

It was demonstrated that CALB immobilized on the 3D printed cross had comparable activity to free CALB. When normalized, the CALB immobilized on the cross demonstrated a 1.8-fold increase in activity for the hydrolysis of p-NPL compared to free CALB.

**Reuse of 3D Scaffold Sintered Materials with** CALB: The 3D printed crosses and beads that were previously immobilized with 100 $\mu$g/mL CALB and 250 $\mu$g/mL NPs were boiled in pH 2 water obtained from a BamStead Nanopure water purifier (Thermo Scientific, 18.2 MOhm-cm) for 20 minutes to remove any enzymes/NPs. To validate that all enzymes and NPs were removed from the crosses and beads, the previously described colorimetric assay of glycerol ester hydrolysis was used. The assay measured the hydrolysis of para-nitrophenyl laurate (p-NPL) into para-nitrophenol (p-NP) and lauric acid. The p-NPL was purchased from Fisher Scientific (cat. 50-014-39022) and p-NP was purchased from Sigma Aldrich (cat. 48549). A reaction mix was prepared with 100 $\mu$M p-NPL in 50 mM Tris (pH 7.5) (cat. RGF-3350) purchased from KD Medical (Columbia, MD, USA). The reaction mix was added to the immobilized and free enzyme systems then incubated for 4 minutes at 25°C. The supernatant was collected then p-NP was quantified by measuring absorbance at 400 nm and compared to a standard of p-NP.

The washed crosses and beads were used to re-immobilize Lipase B from *Candida antarctica* (CALB) purchased from Sigma Aldrich (cat. 62288). All water was obtained from a BarnStead Nanopure water purifier (Thermo Scientific, 18.2 MOhm-cm). Iron oxide nanoparticles (NPs) were prepared as previously described (U.S. Patent No. 9,597,672; Intl Pub. No. WO2018102319) and stored under a $N_2$ sparged atmosphere at pH 11 at 4°C.

Nanoparticles were ultrasonicated (Fisher Scientific) on the day of immobilization for 1 minute at 40% amplitude. NP solutions of 500 $\mu$g/mL were prepared from a 24 mg/mL stock solution with pH 3 water. Cold NP solution was added rapidly to cold enzyme solution containing: 200 $\mu$g/mL of CALB in pH 7 water. The NP-enzyme mix was then added to a centrifuge tube (Eppendorf) and incubated for 2 hours at 25°C. The immobilization yield was quantified by the Bradford method (Bradford reagent: Quick Start™ Bradford 1x Dye Reagent #5000205 from BioRad) by comparing against CALB enzyme standards.

To assess the activity of immobilized and free CALB, a colorimetric assay of glycerol ester hydrolysis was developed. The assay measured the hydrolysis of para-nitrophenyl laurate (p-NPL) into para-nitrophenol (p-NP) and lauric acid. The p-NPL was purchased from Fisher Scientific (cat. 50-014-39022) and p-NP was purchased from Sigma Aldrich (cat. 48549). A reaction mix was prepared with 100 $\mu$M p-NPL in 50 mM Tris (pH 7.5) (cat. RGF-3350) purchased from KD Medical (Columbia, MD, USA). The reaction mix was added to the immobilized and free enzyme systems, then incubated for 4 minutes at 25 °C. The supernatant was collected, then p-NP was quantified by measuring absorbance at 400 nm and compared to a standard of p-NP.

The relative activity of CALB on the 3D printed cross and bead after washing (in triplicate) were 0% $\pm$ 3% and 0% $\pm$ 2%, respectively (Figure 7A). The immobilization yields of re-immobilized CALB on the cross and bead were 98% ± 4% and 100%, respectively (Figure 7B). The activity of the fresh immobilized CALB was about 2-fold higher than the free enzyme (Figure 7C). The results of the activity assay on the washed crosses and beads indicate that 3D printed scaffolds can be cleared of previously immobilized enzymes and NPs. Furthermore, the scaffolds can be re-functionalized with fresh enzymes with minimal losses to the immobilization yields.

## Example 4 - BM3 P450 Immobilization on Sintered Materials

Recombinant BM3 Cytochrome p450, recombinant glucose dehydrogenase (GDH), Bovine serum albumin (BSA), Bovine liver catalase (CAT), Bovine erythrocyte cytosolic superoxide dismutase (SOD) expressed in *E. coli*, glucose (beta-d-glucose), p-nitrophenyl laurate (p-NPL), p-nitrophenol (p-NP), nicotinamide adenine dinucleotide phosphate (reduced) tetrasodium salt (NADPH), are immobilized on the 3D printed scaffolds. All stock solutions are made with 18.2 MΩ-cm water purified by Bamstead™ Nanopure™ water purifier (Thermo Scientific). Absorbance is measured in triplicate in Costar™ 3635 UV-transparent microplates using Biotek Synergy4™ plate reader operating with Gen5™ software. A sonicator (FB-505) with a ⅛ inch probe from Fisher Scientific® (Waltham, MA) is used for all sonication. Iron oxide nanoparticles (NPs) were prepared as previously described (U.S. Patent No. 9,597,672; Int'l Pub. WO2018102319) and Universal enzyme immobilization within hierarchically-assembled magnetic scaffolds. Catalysis & Biocatalysis 2016;34) and stored under a $N_2$ sparged atmosphere at pH 11 at 4°C.

All aqueous stocks are prepared with ultrapure (MQ) water. Lyophilized BM3-P450, GDH, and NADPH are dissolved in ice-cold oxygen free 2 mM PBS, pH 7.4 and prepared fresh daily. BM3-P450 and GDH are centrifuged at 4°C at 12000g for 10 min to pellet cell debris. Their supernatants are collected, and protein content is quantified using the Bradford assay (Bradford reagent: Quick Start™ Bradford 1x Dye Reagent #5000205 from BioRad) with BSA standards. p-NPL and p-NP stock solutions are prepared in pure DMSO to 100 mM and stored at 4°C. Magnesium chloride (1M) and glucose (100 mM) are dissolved in water and stored at 4°C. All stock solutions are kept on ice. Dilutions are made just before use in assays and allowed to equilibrate to room temperature (21°C).

BM3-P450 immobilizations are optimized using the method described U.S. Patent No. 9,597,672 and Int'l Pub. No. WO2018102319. The non-BM3-P450 biological and chemical component to the immobilization are referred to as the P450 Support System (SS): GDH for cofactor regeneration, CAT/SOD for reactive oxygen species (ROS) control, and NADPH for stability during immobilization. Free BM3-P450 /GDH /CAT/SOD/NADPH stock (500 μg/mL BM3-P450, 100:100:1:1:100 molar ratios) is prepared in cold buffer using fresh enzyme stocks. A 2500 μg/ml NP stock is sonicated at the 40% amplitude for 1 min, equilibrated to room temperature using a water bath, and its pH is adjusted to 3. Free BM3-P450 +SS is dispensed into a microcentrifuge tube to which an equal volume of sonicated NPs is added, then pipette mixed 10 times. The BM3-P450 +SS system is immobilized by adding BM3-P450 +SS to 3D printed scaffolds (crosses and beads) and incubated for 1 hour before it is pelleted magnetically. The immobilization yield is quantified by the Bradford and compared against corresponding enzyme standards.

BM3-P450 activity determination methods are based on BM3-P450 -catalyzed oxidation of p-NPL to form p-NP and ω-1 hydroxylauric acid. Enzyme activity is measured by the increase in absorbance at 410 nm due to the formation of p-NP. BM3-P450 reactions are run at 2°C for 18 hours in microcentrifuge tubes containing 100 mM pH 8.2 phosphate buffered saline (PBS), 0.25 mM p-NPL (0.25% DMSO), 0.15 mM NADPH, 1 mM magnesium chloride, 1 mM glucose, and 3.6 μg/mL CYP (~60 nM). Free enzyme controls also contain 60 nM GDH. The supernatant is collected, then p-NP is quantified by measuring absorbance at 400 nm and compared to a standard of p-NP. One unit (U) of CYP-394 activity is defined as 1 μmol p-NP generated per minute at 21°C in 100 mM PBS (pH 8.2).

The BM3-P450 +SS system is fully immobilized onto 3D printed scaffolds (crosses and beads). The activity is compared to the free enzyme system to show that the 3D printed scaffolds are efficacious in immobilizing and retaining enzymatic activity with multienzyme systems and systems requiring cofactor recycling.

## Example 5 - Invertase Immobilization on 3D Printed Flow Cells

3D printed scaffolds designed for flow cells immobilize invertase. All water is obtained from a BamStead Nanopure water purifier (Thermo Scientific, 18.2 MOhm-cm). Iron oxide nanoparticles (NPs) are prepared as previously described (U.S. Patent No. 9,597,672; Int'l Pub. No. WO2018102319) and stored under a $N_2$ sparged atmosphere at pH 11 at 4°C.

Nanoparticles are ultrasonicated (Fisher Scientific) on the day of immobilization for 1 min at 40% amplitude. NP solutions of 500 μg/mL are prepared from a 24 mg/mL stock solution with pH 3 water. For a 1-gram flow cell, 52 mL of cold NP solution is added rapidly to 52 mL of a cold enzyme solution containing 200 μg/mL of invertase in pH 7 water. The NP-enzyme mix is then added to the 3D printed flow cell and incubated for 2 hours at 25°C. The immobilization yield is quantified by the Bradford method by comparing it to invertase enzyme standards.

The activity of the flow cell is determined by the previously described method of sucrose (So) hydrolysis. The flow cell acts as a packed bed reactor (PBR) and Michaelis-Menten kinetics parameters of $K_m$ = 49 mM and $V_{max}$ = 127

mM/min (Combes et al., Carbohydrate Res. 117(6):215-228 (1983)). The flow rate (F) of reaction mix containing 1 M sucrose in 1X PBS buffer (pH 7.5) is 0.088 mL/min.

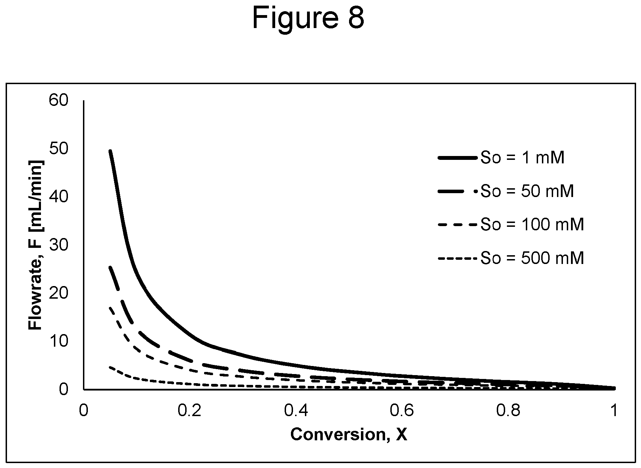

As determined by the Bradford method, the invertase is completely immobilized onto the flow cell with 100% activity being retained. The conversion corresponds to the fundamental PBR mass balance based on a Michaelis-Menten kinetic model to determine the solution space for the flow and loading condition for optimal conversion (Figure 8):

$$V_{max}/F=[S]_0X-K_m*ln(1-X)$$

Given the previous parameters, the conversion (X) of sucrose to glucose and fructose is 99.99%. The residence time is based on the volume in the flow cell, so for a 100 mL reaction mix in a 10 mL flow cell, the residence time is 114 minutes.

The foregoing would indicate that 3D printed scaffolds used as flow cells are efficacious both in terms of immobilization yields and retained activity.

### Example 6 - HRP Immobilization on Sintered Materials

HRP immobilized on sintered materials retains its activity. Horseradish peroxidase (HRP) (cat. 77332) was purchased from Sigma Aldrich (St. Louis, MO, USA). All water was obtained from a BamStead Nanopure water purifier (Thermo Scientific, 18.2 MOhm-cm). Iron oxide nanoparticles (NPs) were prepared as previously described (Corgie SC, Brooks RT, Chairil R, Chun MS, Xie B, Giannelis EP. Universal enzyme immobilisation within hierarchically-assembled magnetic scaffolds. Catalysis & Biocatalysis 2016;34) and stored under a N2 sparged atmosphere at pH 11 at 4 °C.

Nanoparticles were ultrasonicated (Fisher Scientific) on the day of immobilization for 1 min at 40% amplitude. Nanoparticle solutions of 5000 $\mu$g/mL were prepared from a 24 mg/mL stock solution with pH 9 water. Cold NP solution was added rapidly to a cold enzyme solution containing: 1000 $\mu$g/mL of HRP in pH 7 water. The NP-enzyme mix was then added to a centrifuge tube (Eppendorf) along with a 3D-printed strip and incubated for 2 hrs at 25 °C. The immobilization yield was quantified by the Bradford method (Bradford reagent: Quick Start™ Bradford 1x Dye Reagent #5000205 from BioRad) by comparing against HRP enzyme standards.

To assess the activity of immobilized and free HRP, a colorimetric assay of hydrogen peroxide was developed. The assay measured the co-oxidation of 4-aminoantipyrine (4-AAP) and phenol by hydrogen peroxide. The phenol (cat. W322318) and 4-AAP (cat. A4382) was purchased from Sigma Aldrich (St. Louis, MO, USA). A reaction mix was prepared with 1 mM hydrogen peroxide, 0.5 mM 4-AAP, and 0.5 mM phenol in 500 mM PBS (pH 7.4) (cat. SH30258.02) was purchased from Thermo Scientific (Waltham, MA, USA).

The reaction mix was added to the immobilized and free enzyme systems, then incubated for 3 minutes at 25 °C. The supernatant was collected, then the product (pink coloured quinoneimine dye) was quantified by measuring the absorbance at 500 nm.

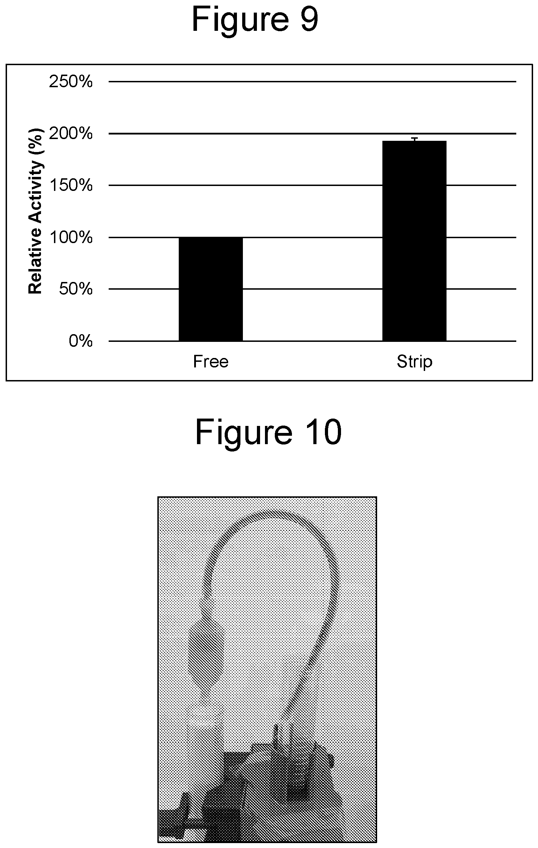

The immobilization yields of HRP on the 3D printed strip (in triplicate) were 95% $\pm$ 1%. The relative activity of immobilized HRP to free enzyme for the co-oxidation of 4-AAP and phenol was 193% $\pm$ 3%, when normalized by the immobilization yield. (Figure 9).

### Example 7 - HRP Immobilization on 3D Printed Flow Cells

Immobilized HRP retains its activity in a 3D printed flow cell as determined by a colorimetric assay. Horseradish peroxidase (HRP) (cat. 77332) was purchased from Sigma Aldrich (St. Louis, MO, USA). All water was obtained from a BamStead Nanopure water purifier (Thermo Scientific, 18.2 MOhm-cm). Iron oxide nanoparticles (NPs) were prepared as previously described (Corgie SC, Brooks RT, Chairil R, Chun MS, Xie B, Giannelis EP. Universal enzyme immobilisation within hierarchically-assembled magnetic scaffolds. Catalysis & Biocatalysis 2016;34) and stored under a N2 sparged atmosphere at pH 11 at 4 °C.

Nanoparticles were ultrasonicated (Fisher Scientific) on the day of immobilization for 1 min at 40% amplitude. Nanoparticle solutions of 5000 $\mu$g/mL were prepared from a 24 mg/mL stock solution with pH 9 water. Cold NP solution was added rapidly to a cold enzyme solution containing: 1000 $\mu$g/mL of HRP in pH 7 water. The NP-enzyme mix was then added to a centrifuge tube (Eppendorf) along with a 3D-printed strip and incubated for 2 hrs at 25 °C. The immobilization yield was quantified by the Bradford method (Bradford reagent: Quick Start™ Bradford 1x Dye Reagent #5000205 from BioRad) by comparing against HRP enzyme standards.

After mixing, the resulting solution was circulated through the flow cell for 1 hour. To assess the activity of immobilized and free HRP, a colorimetric assay of hydrogen peroxide was developed. The assay measured the co-oxidation of 4-aminoantipyrine (4-AAP) and phenol by hydrogen peroxide. The phenol (cat. W322318) and 4-AAP (cat. A4382) was purchased from Sigma Aldrich (St. Louis, MO, USA).

A reaction mix was prepared with 1 mM hydrogen peroxide, 0.5 mM 4-AAP, and 0.5 mM phenol in 500 mM PBS (pH 7.4) (cat. SH30258.02) was purchased from Thermo Scientific (Waltham, MA, USA). The reaction mix was added to the immobilized and free enzyme systems, then incubated for 3 minutes at 25 °C. The supernatant was collected, then the product (pink colored quinoneimine dye) was quantified by measuring the absorbance at 500 nm. Identical reaction mix was pumped through the flow cell via syringe pump (NE-100, New Era Pump Systems Inc., Farmingdale, NY) at 2 mL/min to colorimetrically determine the activity of the immobilized HRP (Figure 10). HRP was successfully immobilized on a 3D printed flow cell and retained activity as indicated by the colorimetric assay.

**Example 8 - Epimerase Immobilization on 3D Printed Flow Cells**

Epimerase was immobilized onto 3D printed scaffolds designed as flow cells. All water was obtained from a BamStead Nanopure water purifier (Thermo Scientific, 18.2 MOhm-cm). Iron oxide nanoparticles (NPs) were prepared as previously described in Corgie, et al., Catalysis & Biocatalysis 34(5): 15-20 (2016). They were stored under a $N_2$ sparged atmosphere at pH 11 at 4 °C.

Nanoparticles were ultrasonicated (Fisher Scientific) on the day of immobilization for 1 min at 40% amplitude. NP solutions of 8.8 mg/mL were prepared from an 8.9 mg/mL stock solution with pH 3 water. For a 1.8 gram flow cell, 26.2 mL of cold NP solution was added rapidly to 26.2 mL of a cold enzyme solution containing: 3.5 mg/mL of epimerase in pH 8 water with 4 mM Tris (pH 7.5) (cat. RGF-3350) purchased from KD Medical (Columbia, MD, USA). The NP-enzyme mix was then circulated through a 6 mL 3D printed flow cell at 5 mL/min and incubated for 2 hr at 25 °C. The immobilization yield was quantified by the Bradford method (Bradford reagent: Quick Start™ Bradford 1x Dye Reagent #5000205 from BioRad) by comparing it against epimerase enzyme standards. The Bradford analysis showed that 87% ± 1% of the epimerase was immobilized onto the flow cell.

Exemplary Sequences

SEQ ID NO:1
Bifunctional P450/NADPH-P450 reductase [*Bacillus megaterium*]

MTIKEMPQPKTFGELKNLPLLNTDKPVQALMKIADELGEIFKFEAPGRVTRYLSSQRL
IKEACDESRFDKNLSQALKFVRDFAGDGLFTSWTHEKNWKKAHNILLPSFSQQAMK
GYHAMMVDIAVQLVQKWERLNADEHIEVPEDMTRLTLDTIGLCGFNYRFNSFYRDQ
PHPFITSMVRALDEAMNKLQRANPDDPAYDENKRQFQEDIKVMNDLVDKIIADRKA
SGEQSDDLLTHMLNGKDPETGEPLDDENIRYQIITFLIAGHETTSGLLSFALYFLVKNP
HVLQKAAEEAARVLVDPVPSYKQVKQLKYVGMVLNEALRLWPTAPAFSLYAKEDT
VLGGEYPLEKGDELMVLIPQLHRDKTIWGDDVEEFRPERFENPSAIPQHAFKPFGNG
QRACIGQQFALHEATLVLGMMLKHFDFEDHTNYELDIKETLTLKPEGFVVKAKSKKI
PLGGIPSPSTEQSAKKVRKKAENAHNTPLLVLYGSNMGTAEGTARDLADIAMSKGF
APQVATLDSHAGNLPREGAVLIVTASYNGHPPDNAKQFVDWLDQASADEVKGVRY
SVFGCGDKNWATTYQKVPAFIDETLAAKGAENIADRGEADASDDFEGTYEEWREH
MWSDVAAYFNLDIENSEDNKSTLSLQFVDSAADMPLAKMHGAFSTNVVASKELQQ
PGSARSTRHLEIELPKEASYQEGDHLGVIPRNYEGIVNRVTARFGLDASQQIRLEAEEE
KLAHLPLAKTVSVEELLQYVELQDPVTRTQLRAMAAKTVCPPHKVELEALLEKQAY
KEQVLAKRLTMLELLEKYPACEMKFSEFIALLPSIRPRYYSISSSPRVDEKQASITVSV
VSGEAWSGYGEYKGIASNYLAELQEGDTITCFISTPQSEFTLPKDPETPLIMVGPGTGV
APFRGFVQARKQLKEQGQSLGEAHLYFGCRSPHEDYLYQEELENAQSEGIITLHTAFS
RMPNQPKTYVQHVMEQDGKKLIELLDQGAHFYICGDGSQMAPAVEATLMKSYADV
HQVSEADARLWLQQLEEKGRYAKDVWAG

SEQ ID NO:2
Cytochrome P450 3A4 isoform 1 [*Homo sapiens*]

MALIPDLAMETWLLLAVSLVLLYLYGTHSHGLFKKLGIPGPTPLPFLGNILSYHKGFC
MFDMECHKKYGKVWGFYDGQQPVLAITDPDMIKTVLVKECYSVFTNRRPFGPVGF
MKSAISIAEDEEWKRLRSLLSPTFTSGKLKEMVPIIAQYGDVLVRNLRREAETGKPVT
LKDVFGAYSMDVITSTSFGVNIDSLNNPQDPFVENTKKLLRFDFLDPFFLSITVFPFLIP
ILEVLNICVFPREVTNFLRKSVKRMKESRLEDTQKHRVDFLQLMIDSQNSKETESHKA

LSDLELVAQSIIFIFAGYETTSSVLSFIMYELATHPDVQQKLQEEIDAVLPNKAPPTYD
TVLQMEYLDMVVNETLRLFPIAMRLERVCKKDVEINGMFIPKGVVVMIPSYALHRD
PKYWTEPEKFLPERFSKKNKDNIDPYIYTPFGSGPRNCIGMRFALMNMKLALIRVLQ
NFSFKPCKETQIPLKLSLGGLLQPEKPVVLKVESRDGTVSGA

SEQ **ID** NO:3
Cytochrome P450 1A2 [*Homo sapiens*]

MALSQSVPFSATELLLASAIFCLVFWVLKGLRPRVPKGLKSPPEPWGWPLLGHVLTL
GKNPHLALSRMSQRYGDVLQIRIGSTPVLVLSRLDTIRQALVRQGDDFKGRPDLYTS
TLITDGQSLTFSTDSGPVWAARRRLAQNALNTFSIASDPASSSSCYLEEHVSKEAKALI
SRLQELMAGPGHFDPYNQVVVSVANVIGAMCFGQHFPESSDEMLSLVKNTHEFVET
ASSGNPLDFFPILRYLPNPALQRFKAFNQRFLWFLQKTVQEHYQDFDKNSVRDITGA
LFKHSKKGPRASGNLIPQEKIVNLVNDIFGAGFDTVTTAISWSLMYLVTKPEIQRKIQ
KELDTVIGRERRPRLSDRPQLPYLEAFILETFRHSSFLPFTIPHSTTRDTTLNGFYIPKKC
CVFVNQWQVNHDPELWEDPSEFRPERFLTADGTAINKPLSEKMMLFGMGKRRCIGE
VLAKWEIFLFLAILLQQLEFSVPPGVKVDLTPIYGLTMKHARCEHVQARLRFSIN

**SEQ ID NO:4**
**CYP2D6 [*Homo sapiens*]**

MGLEALVPLAMIVAIFLLLVDLMHRRQRWAARYPPGPLPLPGLGNLLHVDFQNTPY
CFDQLRRRFGDVFSLQLAWTPVVVLNGLAAVREALVTHGEDTADRPPVPITQILGFG
PRSQGRPFRPNGLLDKAVSNVIASLTCGRRFEYDDPRFLRLLDLAQEGLKEESGFLRE
VLNAVPVLLHIPALAGKVLRFQKAFLTQLDELLTEHRMTWDPAQPPRDLTEAFLAE
MEKAKGNPESSFNDENLCIVVADLFSAGMVTTSTTLAWGLLLMILHPDVQRRVQQEI
DDVIGQVRRPEMGDQAHMPYTTAVIHEVQRFGDIVPLGVTHMTSRDIEVQGFRIPKG
TTLITNLSSVLKDEAVWEKPFRFHPEHFLDAQGHFVKPEAFLPFSAGRRACLGEPLAR
MELFLFFTSLLQHFSFSVPTGQPRPSHHGVFAFLVTPSPYELCAVPR

**SEQ ID NO:5**
**Cytochrome P450-2E1 [*Homo sapiens*]**

MSALGVTVALLVWAAFLLLVSMWRQVHSSWNLPPGPFPLPIIGNLFQLELKNIPKSF
TRLAQRFGPVFTLYVGSQRMVVMHGYKAVKEALLDYKDEFSGRGDLPAFHAHRDR

GIIFNNGPTWKDIRRFSLTTLRNYGMGKQGNESRIQREAHFLLEALRKTQGQPFDPTF
LIGCAPCNVIADILFRKHFDYNDEKFLRLMYLFNENFHLLSTPWLQLYNNFPSFLHYL
PGSHRKAIKNVAEVKEYVSERVKEHHQSLDPNCPRDLTDCLLVEMEKEKHSAERLY
TMDGITVTVADLFFAGTETTSTTLRYGLLILMKYPEIEEKLHEEIDRVIGPSRIPAIKDR
QEMPYMDAVVHEIQRFITLVPSNLPHEATRDTIFRGYLIPKGTVVVPTLDSVLYDNQE
FPDPEKFKPEHFLNENGKFKYSDYFKPFSTGKRVCAGEGLARMELFLLLCAILQHFNL
KPLVDPKDIDLSPIHIGFGCIPPRYKLCVIPRS

**SEQ ID NO:6**
**Cytochrome P450-2E1 [*Homo sapiens*]**

MSALGVTVALLVWAAFLLLVSMWRQVHSSWNLPPGPFPLPIIGNLFQLELKNIPKSF
TRLAQRFGPVFTLYVGSQRMVVMHGYKAVKEALLDYKDEFSGRGDLPAFHAHRDR
GIIFNNGPTWKDIRRFSLTTLRNYGMGKQGNESRIQREAHFLLEALRKTQGQPFDPTF
LIGCAPCNVIADILFRKHFDYNDEKFLRLMYLFNENFHLLSTPWLQLYNNFPSFLHYL
PGSHRKAIKNVAEVKEYVSERVKEHHQSLDPNCPRDLTDCLLVEMEKEKHSAERLY
TMDGITVTVADLFFAGTETTSTTLRYGLLILMKYPEIEEKLHEEIDRVIGPSRIPAIKDR
QEMPYMDAVVHEIQRFITLVPSNLPHEATRDTIFRGYLIPKGTVVVPTLDSVLYDNQE
FPDPEKFKPEHFLNENGKFKYSDYFKPFSTGKRVCAGEGLARMELFLLLCAILQHFNL
KPLVDPKDIDLSPIHIGFGCIPPRYKLCVIPRS

**SEQ ID NO:7**
**Cytochrome P450, family 2, subfamily C, polypeptide 9 [*Homo sapiens*]**

MDSLVVLVLCLSCLLLLSLWRQSSGRGKLPPGPTPLPVIGNILQIGIKDISKSLTNLSK
VYGPVFTLYFGLKPIVVLHGYEAVKEALIDLGEEFSGRGIFPLAERANRGFGIVFSNG
KKWKEIRRFSLMTLRNFGMGKRSIEDRVQEEARCLVEELRKTKASPCDPTFILGCAP
CNVICSIIFHKRFDYKDQQFLNLMEKLNENIKILSSPWIQICNNFSPIIDYFPGTHNKLL
KNVAFMKSYILEKVKEHQESMDMNNPQDFIDCFLMKMEKEKHNQPSEFTIESLENT
AVDLFGAGTETTSTTLRYALLLLLKHPEVTAKVQEEIERVIGRNRSPCMQDRSHMPY
TDAVVHEVQRYIDLLPTSLPHAVTCDIKFRNYLIPKGTTILISLTSVLHDNKEFPNPEM
FDPHHFLDEGGNFKKSKYFMPFSAGKRICVGEALAGMELFLFLTSILQNFNLKSLVDP
KNLDTTPVVNGFASVPPFYQLCFIPV

The foregoing description has been presented only for purposes of illustration and description. This description is not intended to limit the invention to the precise form disclosed. It is intended that the scope of the invention be defined by the claims appended hereto.

**Claims**

1. A shaped magnetic macroporous scaffold formed into said shape by three-dimensional (3D) printing; wherein said shaped magnetic macroporous scaffold comprises:

   a. a magnetic macroporous powder comprising a thermoplastic polymer and magnetic microparticles;
   b. self-assembled mesoporous aggregates of magnetic nanoparticles; and

   i. optionally wherein said self-assembled mesoporous aggregates of magnetic nanoparticles further comprise one or more enzymes magnetically immobilized within said mesopores or on the surface of said magnetic nanoparticles;
   ii. optionally wherein said magnetic particles have a size of: between about 50-100$\mu$m, between about 10-50$\mu$m, between about 5-10$\mu$m, about 10$\mu$m, about 5$\mu$m, less than about 5$\mu$m, or greater than about 100$\mu$m;
   iii. optionally wherein said magnetic macroporous powder has an average size of about 150 $\mu$m, 75 $\mu$m or 15 $\mu$m; or
   iv. optionally wherein said magnetic particles have a concentration of between 0 and 10% by weight, 10 to 50% by weight, or 50 to 90% by weight.

**2.** The shaped magnetic macroporous scaffold of claim 1, wherein said thermoplastic polymer comprises a polymer selected from the group consisting of Polyvinyl alcohol (PVA), Acrylic (PMMA), Acrylonitrile butadiene styrene (ABS), Polyamide including Nylon 6 and Nylon 12, Polylactic acid (PLA), Polybenzimidazole (PBI), Polycarbonate (PC), Polyether sulfone (PES), Polyoxymethylene (POM), Polyetherether ketone (PEEK), Polyetherimide (PEI), Polyethylene (PE), Polyphenylene oxide (PEO), Polyphenylene sulfide (PPS), Polypropylene (PP), Polystyrene (PS), Polyvinyl chloride (PVC), polytetrafluoroethylene (PTFE), co-polyesters, and chemically functionalized derivatives thereof.

**3.** The shaped magnetic macroporous scaffold of claim 1, wherein said magnetic microparticles comprise a magnetic material selected from the group consisting of magnetite ($Fe_3O_4$), hematite ($\alpha$-$Fe_2O_3$), maghemite ($\gamma$-$Fe_2O_3$), a spinel ferrite, lodestone, cobalt, nickel, rare earth, and magnetic composites;

    a. optionally wherein said rare earth is neodymium, gadolinium, sysprosium, samarium-cobalt, or neodymium-iron-boron; or
    b. optionally wherein said magnetic composite comprises a ceramic, ferrite, or alnico magnets.

**4.** The shaped magnetic macroporous scaffold of any one of claims 1-3, wherein said thermoplastic polymer and said magnetic microparticles are chemically, thermally, or physically blended;

    a. optionally wherein the magnetic macroporous powder comprises macropores having a size of between 0.5-200$\mu$m; or
    b. optionally wherein the magnetic macroporous powder further comprises cellulose fibers, cellulose nanofibers, glass fibers, or carbon fibers.

**5.** The shaped magnetic macroporous scaffold of any one of claims 1-4 wherein said shape is a cylinder, an orb, a bead, a strip, a capsule, a cube, a squared rod, a pyramid, a diamond, a lattice, or an irregular shape

**6.** The shaped magnetic macroporous scaffold of any one of claims 1-5 further comprising an enzyme magnetically immobilized within said mesopores or on their surface.

**7.** The shaped magnetic macroporous scaffold of any one of claim 6, wherein said one or more enzymes are selected from the group consisting of hydrolases, hydroxylases, hydrogen peroxide producing enzymes (HPP), nitralases, hydratases, dehydrogenases, transaminases, ketoreductases (KREDS) ene reductases (EREDS), imine reductases (IREDS), catalases, dismutases, oxidases, dioxygenases, lipoxidases, oxidoreductases, peroxidases, laccases, synthetases, transferases, oxynitrilases, isomerases, gludosidases, kinases, lyases, sucrases, invertases, epimerases, and lipases.

**8.** The shaped magnetic macroporous scaffold of claim 1, wherein said self-assembled mesoporous aggregates of magnetic nanoparticles comprise microsomes, wherein a first enzyme requiring a diffusible cofactor having a first enzymatic activity is contained within said microsomes, wherein a second enzyme comprising a cofactor regeneration activity is magnetically-entrapped within said mesopores, wherein said cofactor is utilized in said first enzymatic activity; wherein said first and second enzymes function by converting a diffusible substrate into a diffusible product; and wherein said magnetic nanoparticles are magnetically associated with said magnetic macroporous scaffold.

**9.** The shaped magnetic macroporous scaffold of claim 1, wherein said self-assembled mesoporous aggregates of magnetic nanoparticles comprises a first enzyme requiring a diffusible cofactor having a first enzymatic activity; a second enzyme comprising a cofactor regeneration activity; wherein said cofactor is utilized in said first enzymatic activity; wherein said first and second enzymes are magnetically-entrapped within said mesopores formed by said aggregates of magnetic nanoparticles and said first and second enzymes function by converting a diffusible substrate into a diffusible product;

    a. optionally wherein said first enzyme is an oxidative enzyme;
    b. optionally wherein said oxidative enzyme is a Flavin-containing oxygenase;
    wherein said composition further comprises a third enzyme having a co-factor reductase activity that is co-located with said first enzyme;
    c. optionally wherein said oxidative enzyme is a P450 monooxygenase; wherein said composition further comprises a third enzyme having a co-factor reductase activity that is co-located with said first enzyme;
    d. optionally wherein said P450 monooxygenase and said third enzyme are comprised within a single protein;

e. optionally wherein said single protein comprises a bifunctional cytochrome P450/NADPH--P450 reductase;

f. optionally wherein said single protein has BM3 activity and has at least a 90% sequence identity to SEQ ID NO:1; or

g. optionally wherein said scaffold is formed in a shape suited for a particular biocatalytic process.

10. A method of making the shaped magnetic macroporous scaffold of any one of claims 1-4, comprising additively manufacturing (AM) said shaped magnetic macroporous scaffold using a three-dimensional (3D) printer, wherein said shape is taken from a 3D model;

a. optionally wherein said 3D model is an electronic file;

b. optionally wherein said electronic file is a computer-aided design (CAD) or a stereolithography (STL) file;

c. optionally wherein said AM is Fused Filament Fabrication (FFF) or Selective laser sintering (SLS); or

d. optionally wherein said macropores are formed using a soluble agent selected from the group consisting of a salt, a sugar, or a small soluble polymer, and removing said soluble agent with a solvent.

11. A method of making a device for catalyzing an enzymatic reaction, comprising combining the shaped magnetic macroporous scaffold of claim 1 and an enzyme, wherein said enzyme is magnetically immobilized within said mesopores.

12. A method of catalyzing a reaction between a plurality of substrates, comprising exposing said shaped magnetic macroporous scaffold of claim 11 to said substrates under conditions in which said enzyme catalyzes said reaction between said substrates.

13. The method of claim 12, wherein said reaction is used in the manufacture of a pharmaceutical product, a medicament, a food product, a flavor, a fragrance, a sweetener, an agrochemical, an antimicrobial agent, a toxin, a detergent, a fuel product, a biochemical product, a paper product, or a plastic product.

14. The method of claim 12, wherein said reaction is used in a process for removing a contaminant from a solution;

a. optionally wherein said solution is an aqueous solution, a solvent, or an oil;

b. optionally further comprising the step of removing said mesoporous aggregates and replacing them with a fresh preparation of mesoporous aggregates; or

c. optionally wherein said method is carried out using flow cell and continuous manufacturing.

**Patentansprüche**

1. Geformtes magnetisches makroporöses Gerüst, das durch dreidimensionalen (3D)-Druck zu der Form gebildet worden ist; wobei das geformte magnetische makroporöse Gerüst umfasst:

a. ein magnetisches makroporöses Pulver, umfassend ein thermoplastisches Polymer und magnetische Mikropartikel;

b. selbstassemblierte mesoporöse Aggregate aus magnetischen Nanopartikeln; und

i. wobei gegebenenfalls die selbstassemblierten mesoporösen Aggregate der magnetischen Nanopartikel des Weiteren ein oder mehrere Enzyme umfassen, die magnetisch in den Mesoporen oder auf der Oberfläche der magnetischen Nanopartikel immobilisiert sind;

ii. wobei gegebenenfalls die magnetischen Partikel eine Größe zwischen etwa 50 und 100 pm, zwischen etwa 10 und 50 pm, zwischen etwa 5 und 10 pm, etwa 10 pm, etwa 5 pm, weniger als etwa 5 $\mu$m oder größer als etwa 100 $\mu$m haben;

iii. wobei gegebenenfalls das magnetische makroporöse Pulver eine durchschnittliche Größe von etwa 150 pm, 75 $\mu$m oder 15 $\mu$m hat; oder

iv. wobei gegebenenfalls die magnetischen Partikel eine Konzentration zwischen 0 und 10 Gew.%, von 10 bis 50 Gew.% oder 50 bis 90 Gew.% haben.

2. Geformtes magnetisches makroporöses Gerüst nach Anspruch 1, wobei das thermoplastische Polymer ein Polymer ausgewählt aus der Gruppe bestehend aus Polyvinylalkohol (PVA), Acrylverbindung (PMMA), Acrylnitril-Butadien-Styrol (ABS), Polyamid einschließlich Nylon 6 und Nylon 12, Polymilchsäure (PLA), Polybenzimidazol (PBI), Polycarbonat (PC), Polyethersulfon (PES), Polyoxymethylen (POM), Polyetheretherketon (PEEK), Polyetherimid (PEI), Polyethylen (PE) Polyphenylenoxid (PEO), Polyphenylensulfid (PPS), Polypropylen (PP), Polystyrol (PS), Polyvinylchlorid (PVC), Polytetrafluorethylen (PTFE), Copolyestern und chemisch funktionalisierten Derivaten davon umfasst.

3. Geformtes magnetisches makroporöses Gerüst nach Anspruch 1, wobei die magnetischen Mikropartikel ein magnetisches Material ausgewählt aus der Gruppe bestehend aus Magnetit ($Fe_3O_4$), Hämatit ($\alpha$-$Fe_2O_3$), Maghemit ($\gamma$-$Fe_2O_3$), einem Spinellferrit, Magnetstein, Kobalt, Nickel, Seltenerde und magnetischen Verbundmaterialien umfasst;

    a. wobei die Seltenerde gegebenenfalls Neodym, Gadolinium, Dysprosium, Samarium-Kobalt oder Neodym-Eisen-Bor ist; oder
    b. wobei der magnetische Verbundstoff gegebenenfalls eine Keramik, Ferrit oder AlNiCo-Magnete umfasst.

4. Geformtes magnetisches makroporöses Gerüst nach einem der Ansprüche 1-3, wobei das thermoplastische Polymer und die magnetischen Mikropartikel chemisch, thermisch oder physikalisch vermischt sind;

    a. wobei gegebenenfalls das magnetische makroporöse Pulver Makroporen mit einer Größe zwischen 0,5 und 200 $\mu$m umfasst; oder
    b. wobei gegebenenfalls das magnetische makroporöse Pulver des Weiteren Cellulosefasern, Cellulosenanofasern, Glasfasern oder Kohlefasern umfasst.

5. Geformtes magnetisches makroporöses Gerüst nach einem der Ansprüche 1-4, wobei die Form ein Zylinder, eine Kugel, eine Perle, ein Streifen, eine Kapsel, ein Kubus, ein Quadratstab, eine Pyramide, eine Raute, ein Gitter oder eine unregelmäßige Form ist.

6. Geformtes magnetisches makroporöses Gerüst nach einem der Ansprüche 1-5, des Weiteren umfassend ein Enzym, das magnetisch in den Mesoporen oder auf deren Oberfläche immobilisiert ist.

7. Geformtes magnetisches makroporöses Gerüst nach einem von Anspruch 6, wobei das eine oder die mehreren Enzyme ausgewählt ist/sind aus der Gruppe bestehend aus Hydrolasen, Hydroxylasen, Wasserstoffperoxid produzierenden Enzymen (HPP), Nitralasen, Hydratasen, Dehydrogenasen, Transaminasen, Ketoreduktasen (KREDS), En-Reduktasen (EREDS), Imin-Reduktasen (IREDS), Katalasen, Dismutasen, Oxidasen, Dioxygenasen, Lipoxidasen, Oxidoreduktasen, Peroxidasen, Laccasen, Synthetasen, Transferasen, Oxynitrilasen, Isomerasen, Gludosidasen, Kinasen, Lyasen, Sucrasen, Invertasen, Epimerases und Lipasen.

8. Geformtes magnetisches makroporöses Gerüst nach Anspruch 1, wobei die selbstassemblierten mesoporösen Aggregate von magnetischen Nanopartikeln Mikrosomen umfassen, wobei ein erstes Enzym, das einen diffusionsfähigen Cofaktor erfordert, mit einer ersten enzymatischen Aktivität in den Mikrosomen enthalten ist, wobei ein zweites Enzym, das eine Cofaktorregenerierungsaktivität umfasst, magnetisch in den Mesoporen eingeschlossen ist, wobei der Cofaktor in der ersten enzymatischen Aktivität genutzt wird; wobei das erste und das zweite Enzym funktionieren, indem ein diffusionsfähiges Substrat in ein diffusionsfähiges Produkt umgewandelt wird; und wobei die magnetischen Nanopartikel magnetisch mit dem magnetischen makroporösen Gerüst assoziiert sind.

9. Geformtes magnetisches makroporöses Gerüst nach Anspruch 1, wobei die selbstassemblierten mesoporösen Aggregate von magnetischen Nanopartikeln umfassen: ein erstes Enzym, das einen diffusionsfähigen Cofaktor erfordert, mit einer ersten enzymatischen Aktivität; ein zweites Enzym, umfassend eine Cofaktorregenerierungsaktivität; wobei der Cofaktor in der ersten enzymatischen Aktivität genutzt wird; wobei das erste und das zweite Enzym magnetisch in den Mesoporen eingeschlossen sind, die durch die Aggregate der Nanopartikel gebildet sind, und das erste und das zweite Enzym funktionieren, indem ein diffusionsfähiges Substrat in ein diffusionsfähiges Produkt umgewandelt wird;

    a. wobei gegebenenfalls das erste Enzym ein oxidierendes Enzym ist;
    b. wobei das oxidierende Enzym gegebenenfalls eine Flavin-haltige Oxygenase ist; wobei die Zusammensetzung des Weiteren ein drittes Enzym mit einer Cofaktorreduktaseaktivität umfasst, das gemeinsam mit dem ersten Enzym verortet ist;
    c. wobei das oxidierende Enzym gegebenenfalls eine P450-Monooxygenase ist; wobei die Zusammensetzung des Weiteren ein drittes Enzym mit einer Cofaktorreduktaseaktivität umfasst, das gemeinsam mit dem ersten Enzym verortet ist;

EP 3 856 900 B1

d. wobei die P450-Monooxygenase und das dritte Enzym gegebenenfalls in einem einzigen Protein enthalten sind;

e. wobei das einzige Protein gegebenenfalls eine bifunktionale Cytochrom P450/NADPH--P450 -Reduktase umfasst;

f. wobei das einzige Protein gegebenenfalls BM3-Aktivität hat und mindestens 90 % Sequenzidentität mit SEQ ID NO:1 hat; oder

g. wobei das Gerüst gegebenenfalls zu einer Form gebildet ist, die für einen speziellen biokatalytischen Prozess geeignet ist.

10. Verfahren zur Herstellung des geformten magnetischen makroporösen Gerüsts nach einem der Ansprüche 1-4, umfassend additive Fertigung (AM) des geformten magnetischen makroporösen Gerüsts unter Verwendung eines dreidimensionalen (3D) Druckers, wobei die Form aus einem 3D-Modell genommen wird;

a. wobei das 3D-Modell gegebenenfalls eine elektronische Datei ist;

b. wobei die elektronische Datei gegebenenfalls eine computergestütztes Design- (CAD) oder eine Stereolithographie- (STL)-Datei ist;

c. wobei gegebenenfalls die AM Fused Filament Fabrication (FFF) oder selektives Lasersintern (SLS) ist; oder

d. wobei die Makroporen gegebenenfalls unter Verwendung eines löslichen Mittels ausgewählt aus der Gruppe bestehend aus einem Salz, einem Zucker oder einem kleinen löslichen Polymer und Entfernen des löslichen Mittels mit einem Lösungsmittel gebildet sind.

11. Verfahren zur Herstellung einer Vorrichtung zum Katalysieren einer enzymatischen Reaktion, umfassend Kombinieren des geformten magnetischen makroskopischen Gerüsts von Anspruch 1 mit einem Enzym, wobei das Enzym in den Mesoporen magnetisch immobilisiert ist.

12. Verfahren zum Katalysieren einer Reaktion zwischen einer Vielzahl von Substraten, umfassend Aussetzen des geformten magnetischen makroporösen Gerüsts nach Anspruch 11 den Substraten unter Bedingungen, unter denen das Enzym die Reaktion zwischen den Substraten katalysiert.

13. Verfahren nach Anspruch 12, wobei die Reaktion zur Fertigung eines pharmazeutischen Produkts, eines Medikaments, eines Nahrungsmittelprodukts, eines Aromas, eines Duftstoffs, eines Süßungsmittels, einer Agrochemikalie, eines antimikrobiellen Mittels, eines Toxins, eines Detergens, eines Brennstoffprodukts, eines biochemischen Produkts, eines Papierprodukts oder eines Kunststoffprodukts verwendet wird.

14. Verfahren nach Anspruch 12, wobei die Reaktion in einem Prozess zum Entfernen einer Verunreinigung aus einer Lösung verwendet wird;

a. wobei die Lösung gegebenenfalls eine wässrige Lösung, ein Lösungsmittel oder ein Öl ist;

b. des Weiteren gegebenenfalls umfassend den Schritt des Entfernens der mesoporösen Aggregate und des Ersetzens derselben durch eine frische Zubereitung von mesoporösen Aggregaten; oder

c. wobei das Verfahren gegebenenfalls unter Verwendung von Durchflusszelle und kontinuierlicher Fertigung durchgeführt wird.


**Revendications**

1. Ossature magnétique macroporeuse mise en forme façonnée dans ladite forme par impression tridimensionnelle (3D), ladite ossature magnétique macroporeuse mise en forme comprenant :

a. une poudre magnétique macroporeuse comprenant un polymère thermoplastique et des microparticules magnétiques ;

b. des agrégats mésoporeux auto-assemblés de nanoparticules magnétiques ; et

i. éventuellement dans laquelle lesdits agrégats mésoporeux auto-assemblés de nanoparticules magnétiques comprennent en outre une ou plusieurs enzymes immobilisées magnétiquement à l'intérieur desdits mésopores ou sur la surface desdites nanoparticules magnétiques ;

ii. éventuellement dans laquelle lesdites particules magnétiques ont une taille : comprise entre environ 50 et 100 pm, comprise entre environ 10 et 50 pm, comprise entre environ 5 et 10 pm, d'environ 10 pm, d'environ 5 pm, inférieure à environ 5 pm, ou supérieure à environ 100 $\mu$m ;

iii. éventuellement dans laquelle ladite poudre magnétique macroporeuse a une taille moyenne d'environ 150 pm, 75 $\mu$m ou 15 $\mu$m ; ou

iv. éventuellement dans laquelle lesdites particules magnétiques ont une concentration comprise entre 0 et 10 % en poids, 10 et 50 % en poids, ou 50 et 90 % en poids.

2. Ossature magnétique macroporeuse mise en forme selon la revendication 1, dans laquelle ledit polymère thermoplastique comprend un polymère choisi dans le groupe constitué par l'alcool polyvinylique (PVA), l'acrylique (PMMA), l'acrylonitrile-butadiène-styrène (ABS), les polyamides, y compris le nylon 6 et le nylon 12, l'acide polylactique (PLA), le polybenzimidazole (PBI), le polycarbonate (PC), la polyéthersulfone (PES), le polyoxyméthylène (POM), la polyétheréthercétone (PEEK), le polyétherimide (PEI), le polyéthylène (PE), l'oxyde de polyphénylène (PEO), le sulfure de polyphénylène (PPS), le polypropylène (PP), le polystyrène (PS), le polychlorure de vinyle (PVC), le polytétrafluoroéthylène (PTFE), les copolyesters, et les dérivés chimiquement fonctionnalisés de ceux-ci.

3. Ossature magnétique macroporeuse mise en forme selon la revendication 1, dans laquelle lesdites microparticules magnétiques comprennent un matériau magnétique choisi dans le groupe constitué par la magnétite ($Fe_3O_4$), l'hématite ($\alpha$-$Fe_2O_3$), la maghémite ($\gamma$-$Fe_2O_3$), une ferrite de type spinelle, l'aimant naturel, le cobalt, le nickel, les terres rares, et les composites magnétiques ;

   a. éventuellement dans laquelle ladite terre rare est le néodyme, le gadolinium, le dysprosium, le samarium-cobalt, ou le néodyme-fer-bore ; ou

   b. éventuellement dans laquelle ledit composite magnétique comprend une céramique, de la ferrite, ou des aimants Alnico.

4. Ossature magnétique macroporeuse mise en forme selon l'une quelconque des revendications 1 à 3, dans laquelle ledit polymère thermoplastique et lesdites microparticules magnétiques sont mélangés chimiquement, thermiquement, ou physiquement ;

   a. éventuellement dans laquelle la poudre magnétique macroporeuse comprend des macropores ayant une taille comprise entre 0,5 et 200 $\mu$m ; ou

   b. éventuellement dans laquelle la poudre magnétique macroporeuse comprend en outre des fibres de cellulose, des nanofibres de cellulose, des fibres de verre, ou des fibres de carbone.

5. Ossature magnétique macroporeuse mise en forme selon l'une quelconque des revendications 1 à 4 dans laquelle ladite forme est un cylindre, une boule, une bille, une bande, une capsule, un cube, une tige carrée, une pyramide, un losange, un réseau, ou une forme irrégulière.

6. Ossature magnétique macroporeuse mise en forme selon l'une quelconque des revendications 1 à 5 comprenant en outre une enzyme immobilisée magnétiquement à l'intérieur desdits mésopores ou sur leur surface.

7. Ossature magnétique macroporeuse mise en forme selon la revendication 6, dans laquelle ladite ou lesdites enzymes sont choisies dans le groupe constitué par les hydrolases, les hydroxylases, les enzymes produisant du peroxyde d'hydrogène (HPP), les nitrilases, les hydratases, les déshydrogénases, les transaminases, les cétoréductases (KREDS), les ène-réductases (EREDS), les imine-réductases (IREDS), les catalases, les dismutases, les oxydases, les dioxygénases, les lipoxydases, les oxydoréductases, les peroxydases, les laccases, les synthétases, les transférases, les oxynitrilases, les isomérases, les glucosidases, les kinases, les lyases, les sucrases, les invertases, les épimérases, et les lipases.

8. Ossature magnétique macroporeuse mise en forme selon la revendication 1, dans laquelle lesdits agrégats mésoporeux auto-assemblés de nanoparticules magnétiques comprennent des microsomes, dans laquelle une première enzyme nécessitant un cofacteur diffusible ayant une première activité enzymatique est contenue à l'intérieur desdits microsomes, dans laquelle une deuxième enzyme présentant une activité de régénération de cofacteur est piégée magnétiquement à l'intérieur desdits mésopores, dans laquelle ledit cofacteur est utilisé dans ladite première activité enzymatique, dans laquelle lesdites première et deuxième enzymes fonctionnent en transformant un substrat diffusible en un produit diffusible, et dans laquelle lesdites nanoparticules magnétiques sont associées magnétiquement à ladite ossature magnétique macroporeuse.

9. Ossature magnétique macroporeuse mise en forme selon la revendication 1, dans laquelle lesdits agrégats mésoporeux auto-assemblés de nanoparticules magnétiques comprennent une première enzyme nécessitant un cofacteur diffusible ayant une première activité enzymatique, une deuxième enzyme présentant une activité de régénération de cofacteur, dans laquelle ledit cofacteur est utilisé dans ladite première activité enzymatique, dans laquelle lesdites première et deuxième enzymes sont piégées magnétiquement à l'intérieur desdits mésopores formés par lesdits agrégats de nanoparticules magnétiques et lesdites première et deuxième enzymes fonctionnent en transformant un substrat diffusible en un produit diffusible ;

    a. éventuellement dans laquelle ladite première enzyme est une enzyme oxydante ;
    b. éventuellement dans laquelle ladite enzyme oxydante est une oxygénase contenant de la flavine, ladite composition comprenant en outre une troisième enzyme ayant une activité de réductase de cofacteur qui est colocalisée avec ladite première enzyme ;
    c. éventuellement dans laquelle ladite enzyme oxydante est une monooxygénase P450, ladite composition comprenant en outre une troisième enzyme ayant une activité de réductase de cofacteur qui est colocalisée avec ladite première enzyme ;
    d. éventuellement dans laquelle ladite monooxygénase P450 et ladite troisième enzyme sont comprises à l'intérieur d'une unique protéine ;
    e. éventuellement dans laquelle ladite unique protéine comprend une cytochrome P450/NADPH-P450 réductase bifonctionnelle ;
    f. éventuellement dans laquelle ladite unique protéine a une activité BM3 et a une identité de séquence d'au moins 90 % avec SEQ ID No 1 ; ou
    g. éventuellement dans laquelle ladite ossature est façonnée dans une forme adaptée à un processus biocatalytique particulier.

10. Procédé de fabrication de l'ossature magnétique macroporeuse mise en forme selon l'une quelconque des revendications 1 à 4, comprenant la fabrication additive (AM) de ladite ossature magnétique macroporeuse mise en forme au moyen d'une imprimante tridimensionnelle (3D), ladite forme étant choisie à partir d'un modèle 3D ;

    a. éventuellement dans lequel ledit modèle 3D est un fichier électronique ;
    b. éventuellement dans lequel ledit fichier électronique est un fichier de conception assistée par ordinateur (CAD) ou de stéréolithographie ;
    c. éventuellement dans lequel ladite AM est le dépôt de filament fondu (FFF) ou le frittage sélectif par laser (SLS) ; ou
    d. éventuellement dans lequel lesdits macropores sont formés en utilisant un agent soluble choisi dans le groupe constitué par un sel, un sucre et un petit polymère soluble, et en retirant ledit agent soluble avec un solvant.

11. Procédé de fabrication d'un dispositif destiné à catalyser une réaction enzymatique, comprenant la combinaison de l'ossature magnétique macroporeuse mise en forme selon la revendication 1 et d'une enzyme, ladite enzyme étant immobilisée magnétiquement à l'intérieur desdits mésopores.

12. Procédé de catalyse d'une réaction entre une pluralité de substrats, comprenant l'exposition de ladite ossature magnétique macroporeuse mise en forme selon la revendication 11 auxdits substrats dans des conditions dans lesquelles ladite enzyme catalyse ladite réaction entre lesdits substrats.

13. Procédé selon la revendication 12, dans lequel ladite réaction est utilisée dans la fabrication d'un produit pharmaceutique, d'un médicament, d'un produit alimentaire, d'un arôme, d'un parfum, d'un édulcorant, d'un produit agrochimique, d'un agent antimicrobien, d'une toxine, d'un détergent, d'un produit carburant, d'un produit biochimique, d'un produit papetier, ou d'un produit plastique.

14. Procédé selon la revendication 12, dans lequel ladite réaction est utilisée dans un processus destiné à retirer un contaminant d'une solution ;

    a. éventuellement dans lequel ladite solution est une solution aqueuse, un solvant, ou une huile ;
    b. éventuellement, comprenant en outre l'étape de retrait desdits agrégats mésoporeux et de remplacement de ceux-ci par une préparation fraîche d'agrégats mésoporeux ; ou
    c. éventuellement dans lequel ledit procédé est réalisé au moyen d'une cellule d'écoulement et d'une fabrication continue.

Figure 1A

Figure 1B

Figure 1C

# Figure 2A

# Figure 2B

# Figure 3

## Figure 4A

30 mm

## Figure 4B

3 mm

8 mm

## Figure 4C

12.7 mm

## Figure 4D

Designs

Figure 4E                    Figure 4F

30 mm

3 mm

8 mm

12.7 mm

Figure 4G                    Figure 4H

3D Prints

## Figure 4I

25 mm

## Figure 4J

25 mm

# Figure 5A

# Figure 5B

# Figure 6A

# Figure 6B

## Figure 7A

## Figure 7B

## Figure 7C

# Figure 8

# Figure 9

# Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62737910
- WO 2014055853 A
- WO 2017180383 A
- EP 3159141 A
- WO 2012122437 A

- WO 2013170050 A
- US 9597672 B
- WO 2018102319 A

### Non-patent literature cited in the description

- **CORGIE et al.** *Chem. Today,* 2016, vol. 34 (5), 15-20
- **KRUTH et al.** *Assembly Automation,* 2003, vol. 23 (4), 357-371
- **SHISHKOVSKY et al.** *Microelectronic Engineering,* 2015, vol. 146, 85-91
- **WEGNER.** *Physics Procedia,* 2016, vol. 83, 1003-1012
- **GOODRIDGE et al.** *Materials Science,* 2012, vol. 57, 229-267
- **KUMAR.** *JOM,* 2003, vol. 55 (10), 43-47
- **SINGH et al.** Microbial enzymes: industrial progress in 21st century. *3 Biotech.,* 2016, vol. 6 (2), 174
- **MOHAMAD et al.** *Biotechnology, Biotechnological Equipment,* 2015, vol. 29 (2), 205-220
- **WILES C et al.** *Green Chem.,* 2012, 38-54
- **TAMBORINI et al.** *Cell,* 2018, vol. 36 (1), 73-78
- **NEWMAN ; JENSEN.** *Green Chem.,* 2013, vol. 15, 1456-1472
- **DICOSIMO et al.** *Chem. Soc. Rev.,* 2013, vol. 42, 6437-6474
- **ANASTAS, P.T.** Handbook of Green Chemistry. Wiley-VCH-Verlag, 2009
- Green chemistry in the pharmaceutical industry. John Wiley & Sons, 2010
- **MARTINEZ et al.** *Curr. Topics Med. Chem.,* 2010, vol. 13 (12), 1470-90
- **WELLS et al.** *Organic Process Res. Dev.,* 2012, vol. 16 (12), 1986-1993
- **GONG et al.** *Microbial Cell Factories,* 2012, vol. 11 (1), 142
- *Br. J. Pharmacol.,* 2009, vol. 158 (1), S215-S217
- **DUAN et al.** *ChemPhysChem,* 2014, vol. 15 (5), 974-980
- **BOSCH et al.** *J.Applied Microbiol.,* 2000, vol. 89 (2), 215-24

- **WELK et al.** *Archives of Oral Biology,* 2011, 2587
- **PURDY, TENOVUO et al.** *Infection and Immunity,* 1983, vol. 39 (3), 1187
- **BOSCH et al.** *J.Applied Microbiol.,* 2000, vol. 89 (2), 215-24
- **WAN, WANG et al.** *Biochemistry Journal,* 2001, vol. 362, 355-362
- **MATHEW ; YUN.** *ACS Catalysis,* 2012, vol. 2 (6), 993-1001
- **SCHÄTZLE et al.** *Anal. Chem.,* 2009, vol. 81 (19), 8244-48
- **GLIEDER et al.** *Chem. Int. Ed.,* 2003, vol. 42, 4815
- **DADASHIPOUR ; ASANO.** ACS Catal. 2011, vol. 1, 1121-49
- **AEHLE W.** Enzymes in Industry,. Weiley-VCH Verlag, GmbH, 2008
- **LINDSKOG ; SILVERMAN et al.** The catalytic mechanism of mammalian carbonic anhydrases EXS. 2000, vol. 90, 175-195
- The Carbonic Anhydrases: New Horizons. 2000, 175-95
- **MCCALL et al.** *J. Nutrition,* 2000, vol. 130, 1455-1458
- **BOONE et al.** *Int'l J. Chem. Engineering,* 2013, vol. 2013, 22-27
- **SHARMA.** *J. Materials Engin. and Performance,* 2003, vol. 12, 324-330
- **COMBES et al.** *Carbohydrate Res.,* 1983, vol. 117 (6), 215-228
- **CORGIE SC ; BROOKS RT ; CHAIRIL R ; CHUN MS ; XIE B ; GIANNELIS EP.** Universal enzyme immobilisation within hierarchically-assembled magnetic scaffolds. *Catalysis & Biocatalysis,* 2016, vol. 34
- **CORGIE et al.** *Catalysis & Biocatalysis,* 2016, vol. 34 (5), 15-20